# EUROPEAN PATENT APPLICATION

(11) **EP 4 242 203 A1**
(43) Date of publication of application: **13.09.2023**
(21) Application number: 21889635.5
(22) Date of filing: 05.11.2021
(51) Int. Cl.: C07D 401/12, A61K 31/5377, A61K 31/506, A61P 31/20, A61P 31/14, A61P 31/18, C07D 239/48

(54) **NOVEL CAPSID ASSEMBLY INHIBITOR**

(30) Priority: 06.11.2020 KR 20200147948
(71) Applicant: Pelemed Co., Ltd., Gwangju 61005 (KR)
(72) Inventor: KIM, Yong Chul, Gwangju 61005 (KR); PARK, Sung Gyoo, Gwangju 61005 (KR); SEO, Jiwon, Gwangju 61005 (KR); KIM, Woo Chan, Gwangju 61005 (KR); JUNG, Jae Hoon, Gwangju 61005 (KR); CHO, Yuri, Seoul 06603 (KR); KIM, Yoon Jun, Seoul 06549 (KR)
(74) Representative: Vossius & Partner Patentanwälte Rechtsanwälte mbB
(86) International application number: PCT/KR2021/016068
(87) International publication number: WO 2022/098170

(57) **Abstract**

The present invention relates to a series of novel phenylaminopyridine derivatives and a use thereof for inhibiting capsid assembly and for preventing or treating virus infectious diseases thereby.

## Description

### TECHNICAL FIELD

The present invention relates to a series of novel phenylaminopyrimidine derivatives, and specifically, to prevention or treatment of viral infectious diseases through capsid assembly inhibitory activity of the derivatives.

### BACKGROUND ART

Chronic hepatitis B virus (HBV) infection is a major health problem worldwide and may lead to serious health issues such as cirrhosis or liver cancer. According to a recent WHO report, in 2015, it was estimated that 257 million people worldwide lived with chronic HBV, and 1.34 million deaths were caused by hepatitis-related complications. Up to now, substances approved for treating HBV include interferons (IFNs, non-pegylated or pegylated), nucleos(t)ide analogues, lamivudine, adefovir, entecavir and tenofovir, etc. IFNs therapy inhibits HBV replication and induces remission of liver disease, and nucleos(t)ide drugs inhibit activity of reverse transcriptase and DNA polymerase. Nevertheless, until today complete elimination of HBV using antiviral drugs is difficult, and drug resistance caused by long-term use of antiviral drugs frequently occurs. To overcome these unmet medical needs, discovery of efficient and safe anti-HBV drugs with novel molecular targets is required.

HBV viral genomes are enclosed by a HBV capsid, which is assembled in the form of a core protein. The HBV capsid not only protects DNA but is also associated with pregenomicRNA (pgRNA) encapsidation. In addition, it regulates transport and nuclear release of the viral genomes, and participates in epigenetic regulation along with ccc DNA, and modulates host gene expression, reverse transcription of pgRNA, and recycling of nucleocapsids. As such, based on the HBV replication cycle, discovery of anti-HBV agents targeting the capsid protein has been extensively studied.

Several research groups and pharmaceutical companies are developing capsid assembly regulators. Bay-41-4109, a heteroaryldihydropyrimidine (HAP) analogue, is the first capsid assembly inhibitor which entered into clinical trial, inducing aberrant formation of the capsid and aggregation of the capsid protein. As a result of incorrect assembly of the capsid protein, proteasome-mediated degradation of HBV core proteins along with HBV DNA reduction was observed in HepG2.2.15 cells. GLS-4 is a second-generation HAP analogue having the same mechanism of action as BAY-41-4109 and is being studied in phase II clinical trial along with ritonavir (RTV) for preventing CYP induction by GLS-4. Recently, the same research groups reported HEC72702 showing improved oral bioavailability, despite reduced induction of CYP enzymes, low inhibition of hERG K+ channels and *in vitro* potency reduced by 2 to 3 times compared to 2. Along with other capsid assembly regulators, AT130, NVR 3-778 and JNJ-632, which have a different mechanism of action than HAPs, have been developed. For example, treatment of NVR 3-778, despite binding to capsid proteins, still promoted the process of normal-sized capsid assembly, while inducing empty capsid formation. JNJ-632 was reported as a novel sulfamoylbenzamide capsid assembly regulator, and its optimized analogue JNJ-6379 is currently being studied in a phase II clinical trial. Recently, the present inventors reported a ciclopirox, a FDA-approved antifungal drug, as an orally available capsid assembly inhibitor as a drug repositioning strategy.

### DETAILED DESCRIPTION

### TECHNICAL OBJECTIVE

As a result of intensive research to discover novel small molecule compounds capable of blocking viral infection by inhibiting capsid assembly, the inventors completed the present invention by discovering that a series of phenylaminopyrimidine derivatives has an activity of inhibiting viral infections such as hepatitis B virus infection, by potentially inhibiting capsid assembly.

### WORKING EFFECT OF THE INVENTION

The phenylaminopyrimidine derivatives in the newly synthesized molecules according to the present invention exhibit an inhibitory effect on capsid assembly, making them useful for preventing or treating diseases related thereto, e.g., viral infectious diseases such as HBV, HCV and HIV.

### BEST MODE FOR CARRYING OUT THE INVENTION

Hereinafter the present invention will be discussed in detail. Meanwhile, each description and embodiment disclosed in the present invention may also be applied to other descriptions and embodiments. That is, all combinations of the various elements disclosed herein are involved in the scope of the present invention. Moreover, the specific descriptions described below shall not restrict the scope of the present invention.

A first aspect of the present invention is to provide a compound represented by Formula 1 below or a pharmaceutically acceptable salt thereof:

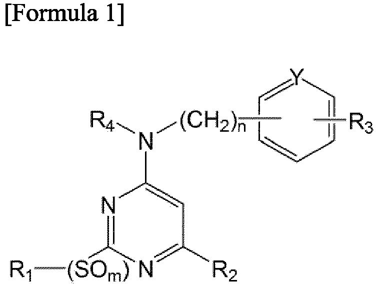

In Formula 1,
m is 0, 1, or 2;
n is an integer from 0 to 4;
Yis C(H) or N;
R₁ is C₁₋₄ alkyl;
R₂ is hydrogen, halogen, amino, -NH-(CH₂)ₚ-NHCO-R₅, or
C₆₋₁₀ aryl, C₆₋₁₀ arylamino, 3-10 membered heterocyclyl, or 3-10 membered heterocyclylamino each unsubstituted or substituted by R₆;
R₅ is tert-butoxy, C₆₋₁₀ aryl or 5-10 membered heteroaryl;
R₆ is amino unsubstituted or substituted by tert-butoxycarbonyl, or
tert-butoxy, C_{6-1O} aryl, or 5-10 membered heteroaryl linked directly or through -CO-,
p is an integer from 1 to 4;
R₃ is hydrogen, 3-10 membered heterocyclyl, cyano, C₁₋₄ alkyl, C₁₋₄ haloalkyl, or 1 to 3 halogens; and
R₄ is hydrogen, or C₆₋₁₀ aryl-C₁₋₄ alkyl;
wherein said aryl, heteroaryl and heterocyclyl are unsubstituted or substituted by one or more selected from halogen, C₁₋₄ alkyl, C₁₋₄ alkoxy, or amino.

The term "heteroaryl" and "heterocyclyl" of the present invention are substituents of an unsaturated or saturated ring structure, respectively, containing at least one heteroatom selected from the group consisting of O, N, or S, wherein the heteroatom may be nitrogen but is not limited thereto. For example, heteroaryl or heterocyclyl in the compounds of the present invention may be a substituent of a ring structure containing 1 to 3 nitrogen atoms but is not limited thereto.

For example, in Formula 1, R₁ may be methyl but is not limited thereto.

For example, in Formula 1, R₂ may be hydrogen, halogen, amino, -NH-(CH₂)₂-NHCO-Rs, or phenylamino, piperazinyl, piperidinyl or piperidinylamino unsubstituted or substituted by R₆, wherein R₅ may be tert-butoxy, phenyl or pyridinyl, and R₆ may be tert-butoxy, phenyl or pyridinyl linked directly or through -CO- but is not limited thereto.

Specifically, in Formula 1, R₂ may be hydrogen, chloro, amino,
piperazinyl, piperidinylamino, or aminopiperidinyl each unsubstituted or substituted with butoxycarbonyl,
phenylamino, (methoxypyridinyl)carbonylpiperazinyl, (methoxypyridinyl)carbonylaminoethylamino each substituted by 1 to 3 halogens selected from fluoro or chloro,
phenylcarbonylaminoethylamino substituted by 1 or 2 halogens selected from fluoro or chloro,
(1R,4R)-2,5-diazabicyclo[2.2.1]heptyl unsubstituted or substituted by butoxycarbonyl,
(1S,4S)-2,5-diazabicyclo[2.2.1]heptyl unsubstituted or substituted by butoxycarbonyl,
2,6-diazaspiro[3.3]heptyl unsubstituted or substituted by butoxycarbonyl, or
3-azabicyclo[3.1.0]hexyl unsubstituted or substituted by butoxycarbonyl or butoxycarbonylamino but is not limited thereto.

For example, in Formula 1, R₃ may be hydrogen, morpholinyl, cyano, methyl, trifluoromethyl, or 1 to 3 substituents selected from the group consisting of fluoro, chloro, bromo, and iodo, but not limited thereto.

For example, in Formula 1, R₄ may be hydrogen, benzyl or phenylethyl but is not limited thereto.

Specifically, the compound may be as follows, but is not limited thereto.
1. 2-methoxy-N-(2-(2-(methylthio)-6-(4-morpholinophenylamino)pyrimidin-4-ylamino)ethyl)nicotinamide,
2. 5-methoxy-N-(2-(2-(methylthio)-6-(4-morpholinophenylamino)pyrimidin-4-ylamino)ethyl)nicotinamide,
3. 4-fluoro-N-(2-(2-(methylthio)-6-(4-morpholinophenylamino)pyrimidin-4-ylamino)ethyl)benzamide,
4. 2,4-dichloro-N-(2-(2-(methylthio)-6-(4-morpholinophenylamino)pyrimidin-4-ylamino)ethyl)benzamide,
5. 2-methoxy-N-(2-(2-(methylsulfinyl)-6-(4-morpholinophenylamino)pyrimidin-4-ylamino)ethyl)nicotinamide,
6. 5-methoxy-N-(2-(2-(methylsulfinyl)-6-(4-morpholinophenylamino)pyrimidin-4-ylamino)ethyl)nicotmamide,
7. 4-fluoro-N-(2-(2-(methylsulfinyl)-6-(4-morpholinophenylamino)pyrimidin-4-ylamino)ethyl)benzamide,
8. 2,4-dichloro-N-(2-(2-(methylsulfinyl)-6-(4-morpholinophenylamino)pyrimidin-4-ylamino)ethyl)benzamide,
9. 2-methoxy-N-(2-(2-(methylthio)-6-(phenylamino)pyrimidin-4-ylamino)ethyl)nicotinamide,
10. N-(2-(6-(4-fluorophenylamino)-2-(methylthio)pyrimidin-4-ylamino)ethyl)-2-methoxynicotinamide,
11. N-(2-(6-(4-chlorophenylamino)-2-(methylthio)pyrimidin-4-ylammo)ethyl)-2-methoxynicotmamide,
12. N-(2-(6-(4-bromophenylamino)-2-(methylthio)pyrimidin-4-ylamino)ethyl)-2-methoxynicotmamide,
13. N-(2-(6-(4-iodophenylamino)-2-(methylthio)pyrimidin-4-ylamino)ethyl)-2-methoxynicotinamide,
14. N-(2-(6-(3,4-difluorophenylamino)-2-(methylthio)pyrimidin-4-ylamino)ethyl)-2-methoxynicotinamide,
15. N-(2-(6-(2-chloro-4-fluorophenylamino)-2-(methylthio)pyrimidin-4-ylamino)ethyl)-2-methoxynicotinamide,
16. 2-methoxy-N-(2-(2-(methylsulfinyl)-6-(phenylamino)pyrimidin-4-ylamino)ethyl)nicotinamide,
17. N-(2-(6-(4-fluorophenylamino)-2-(methylsulfinyl)pyrimidin-4-ylamino)ethyl)-2-methoxynicotinamide,
18. N-(2-(6-(4-chlorophenylamino)-2-(methylsulfinyl)pyrimidin-4-ylamino)ethyl)-2-methoxynicotinamide,
19. N-(2-(6-(4-bromophenylamino)-2-(methylsulfinyl)pyrimidin-4-ylamino)ethyl)-2-methoxynicotinamide,
20. N-(2-(6-(4-iodophenylamino)-2-(methylsulfinyl)pyrimidin-4-ylamino)ethyl)-2-methoxynicotinamide,
21. N-(2-(6-(3,4-difluorophenylamino)-2-(methylsulfinyl)pyrimidin-4-ylamino)ethyl)-2-methoxynicotinamide,
22. N-(2-(6-(3,4-difluorophenylamino)-2-(methylsulfonyl)pyrimidin-4-ylamino)ethyl)-2-methoxynicotinamide,
23. N-(2-(6-(2-chloro-4-fluorophenylamino)-2-(methylsulfinyl)pyrimidin-4-ylamino)ethyl)-2-methoxynicotinamide,
24. N-(2-(6-(2-chloro-4-fluorophenylamino)-2-(methylsulfonyl)pyrimidin-4-ylamino)ethyl)-2-methoxynicotinamide,
25. 6-chloro-N-(4-fluorobenzyl)-2-(methylthio)-N-(4-morpholinophenyl)pyrimidin-4-amine,
   (N-(2-(6-(2-chloro-4-fluorophenylamino)-2-(methylsulfonyl)pyrimidin-4-ylamino)ethyl)-2-methoxynicotinamide)
26. 6-chloro-N-(4-fluorophenethyl)-2-(methylthio)-N-(4-morpholinophenyl)pyrimidin-4-amine,
27. N⁴-(4-fluorobenzyl)-2-(methylthio)-N⁴-(4-morpholinophenyl)pyrimidine-4,6-diamine,
28. N⁴-(4-fluorophenethyl)-2-(methylthio)-N⁴-(4-morpholinophenyl)pyrimidine-4,6-diamine,
29. (2-methoxypyridin-3-yl)(4-(2-(methylthio)-6-(4-morpholinophenylamino)pyrimidin-4-yl)piperazin-1-yl)methanone,
30. (2-methoxypyridin-3-yl)(4-(2-(methylsulfinyl)-6-(4-morpholinophenylamino)pyrimidin-4-yl)piperazin-1-yl)methanone,
31. 6-chloro-N-(2,4-difluorophenyl)-2-(methylsulfonyl)pyrimidin-4-amine,
32. 6-chloro-2-(methylsulfonyl)-N-(3,4,5-trifluorophenyl)pyrimidin-4-amine,
33. N⁴,N⁶-bis(4-fluorophenyl)-2-(methylsulfonyl)pyrimidine-4,6-diamine,
34. 2-(methylsulfonyl)-N⁴,N⁶-bis(3,4,5-trifluorophenyl)pyrimidine-4,6-diamine,
35. N⁴,N⁶-bis(3-chloro-4-fluorophenyl)-2-(methylsulfonyl)pyrimidine-4,6-diamine,
36. tert-butyl 4-(6-(4-fluorophenylamino)-2-(methylsulfonyl)pyrimidin-4-yl)piperazine-1 -carboxylate,
37. tert-butyl 4-(6-(4-fluorobenzylamino)-2-(methylsulfonyl)pyrimidin-4-yl)piperazine-1 -carboxylate,
38. tert-butyl 4-(6-(4-chlorophenylamino)-2-(methylsulfonyl)pyrimidin-4-yl)piperazine-1 -carboxylate,
39. tert-butyl 4-(6-(4-chlorobenzylamino)-2-(methylsulfonyl)pyrimidin-4-yl)piperazine-1 -carboxylate,
40. tert-butyl 4-(6-(4-bromophenylamino)-2-(methylsulfonyl)pyrimidin-4-yl)piperazine-1 -carboxylate,
41. tert-butyl 4-(6-(4-iodophenylamino)-2-(methylsulfonyl)pyrimidin-4-yl)piperazine-1 -carboxylate,
42. tert-butyl 4-(6-(3,4-difluorophenylamino)-2-(methylsulfonyl)pyrimidin-4-yl)piperazine-1 -carboxylate,
43. tert-butyl 4-(2-(methylsulfonyl)-6-(3,4,5-trifluorophenylamino)pyrimidin-4-yl)piperazine-1 -carboxylate,
44. tert-butyl 4-(2-(methylsulfonyl)-6-(3,4,5-trifluorobenzylamino)pyrimidin-4-yl)piperazine-1-carboxylate,
45. N-(4-fluorophenyl)-2-(methylsulfonyl)-6-(piperazin-1-yl)pyrimidin-4-amine,
46. 2-(methylsulfonyl)-6-(piperazin-1-yl)-N-(3,4, 5-trifluorophenyl)pyrimidin-4-amine,
47. N-(3-chloro-4-fluorophenyl)-2-(methylsulfonyl)-6-(piperazin-1-yl)pyrimidin-4-amine,
48. N-(3-bromo-4-fluorophenyl)-2-(methylsulfonyl)-6-(piperazin-1-yl)pyrimidin-4-amine,
49. 2-(methylsulfonyl)-N⁴-(piperidin-4-yl)-N⁶-(3,4,5-trifluorophenyl)pyrimidine-4,6-diamine,
50. N⁴-(3-chloro-4-fluorophenyl)-2-(methylsulfonyl)-N⁶-(piperidin-4-yl)pyrimidine-4,6-diamine,
51. N⁴-(3-bromo-4-fluorophenyl)-2-(methylsulfonyl)-N⁶-(piperidin-4-yl)pyrimidine-4,6-diamine,
52. 6-(4-aminopiperidin-1-yl)-2-(methylsulfonyl)-N-(3,4,5-trifluorophenyl)pyrimidin-4-amine,
53. (6-(4-aminopiperidin-1-yl)-N-(3-chloro-4-fluorophenyl)-2-(methylsulfonyl)pyrimidin-4-amine,
54. 6-(4-aminopiperidin-1 -yl)-N-(3 -bromo-4-fluorophenyl)-2-(methylsulfonyl)pyrimidin-4-amine,
55. 6-((1R,4R)-2,5-diazabicyclo[2.2.1]heptan-2-yl)-N-(3-chloro-4-fluorophenyl)-2-(methylsulfonyl)pyrimidin-4-amine,
56. N-(3-chloro-4-fluorophenyl)-2-(methylsulfonyl)-6-(2,6-diazaspiro[3.3]heptan-2-yl)pyrimidin-4-amine,
57. 6-((1S,4S)-2,5-diazabicyclo[2.2.1]heptan-2-yl)-N-(3-chloro-4-fluorophenyl)-2-(methylsulfonyl)pyrimidin-4-amine, or
58. ((1R,5S,6s)-3-(6-(3-chloro-4-fluorophenylamino)-2-(methylsulfonyl)pyrimidin-4-yl)-3-azabicyclo[3.1.0]hexan-6-amine).

The compound of the present invention may exist in the form of pharmaceutically acceptable salts. An acid addition salt formed by a pharmaceutically acceptable free acid is useful as the salt. The term "pharmacologically acceptable salt" of the present invention means any organic or inorganic addition salt of the compound in which side effects caused by this salt, do not degrade the beneficial efficacy of the compound represented by Formula 1. Such salts have a concentration that is relatively non-toxic and a harmless effect on the patient.

Acid addition salts may be prepared using conventional methods. For example, a compound may be dissolved in an excess of an aqueous acid solution, and then the salt can be precipitated using a water-miscible organic solvent, such as methanol, ethanol, acetone, or acetonitrile. Alternatively, equimolar amounts of the compound and an acid or alcohol (e.g., glycol monomethyl ether) in water may be heated, and the resulting mixture can be evaporated or dried, or the precipitated salt may be suction-filtered.

At this time, organic and inorganic acids may be used as the free acid. Inorganic acids such as hydrochloric acid, phosphoric acid, sulfuric acid, nitric acid, tartaric acid, and others may be used. Organic acids such as methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid, and others may be used, but are not limited thereto.

Furthermore, a pharmaceutically acceptable metal salt may be prepared using a base. Alkali metal salts or alkaline earth metal salts may be prepared, for example, by dissolving a compound in an excess of aqueous alkali metal hydroxide or alkali earth metal hydroxide, filtering the undissolved compound salt, and then evaporating and drying the filtrate. At this time, particularly preparing sodium, potassium or calcium salt is pharmaceutically suitable as the metal salt, but not limited thereto. Additionally, the corresponding silver salt may be obtained by reacting an alkali metal or alkaline earth metal salt with a suitable silver salt (e.g., silver nitrate).

Unless otherwise indicated, the pharmaceutically acceptable salts of the compounds of the present invention may include salts of acidic or basic groups that may present in the compounds of Formula 1. For example, sodium, calcium, and potassium salts of a hydroxy group may be included as pharmaceutically acceptable salts. Other pharmaceutically acceptable salts of an amino group may include hydrobromide, sulfate, hydrogen sulfate, phosphate, hydrogen phosphate, dihydrogen phosphate, acetate, succinate, citrate, tartrate, lactate, mandelate, methanesulfonate (mesylate) and p-toluenesulfonate (tosylate) salts, etc. These salts may be prepared using known methods in the art for preparing a salt.

As for the salt of the phenylaminopyrimidine derivative compounds of the present invention, any salt of the phenylaminopyrimidine derivative compounds, which is a pharmaceutically acceptable salt exhibiting pharmacological activity equivalent to that of the phenylaminopyrimidine derivative compound may be used without limitation.

In addition, the compounds represented by Formula 1 according to the present invention may include not only pharmaceutically acceptable salts thereof, but also solvates such as hydrates that may be prepared therefrom, and all possible stereoisomers without limitation. Solvates and stereoisomers of the compounds represented by Formula 1 may be prepared from the compounds represented by Formula 1 using methods known in the art.

Furthermore, the compounds represented by Formula 1 according to the present invention may be prepared in a crystalline form or an amorphous form. When prepared in a crystalline form, the compounds may be optionally hydrated or solvated. In the present invention, not only stoichiometric hydrates of the compounds represented by Formula 1, but also the compounds containing various amounts of water may be included. Solvates of the compounds represented by Formula 1 according to the present invention may include both stoichiometric solvates and non-stoichiometric solvates.

A second aspect of the present invention is a method for preparing a compound represented by Formula 1 or a pharmaceutically acceptable salt thereof. The method may comprise Step 1: preparing a compound represented by Formula 4-1 by reacting a compound represented by Formula 2-1 with an amine derivative represented by Formula 3; and
optionally, when R₄ is not hydrogen, Step 2: preparing a compound represented by Formula 6 by reacting a compound represented by Formula 4-1 with a halide derivative represented by Formula 5.

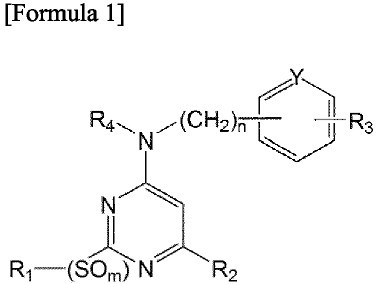

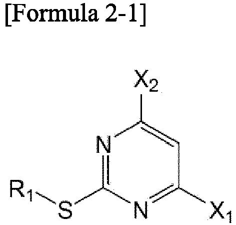

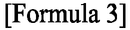

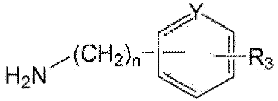

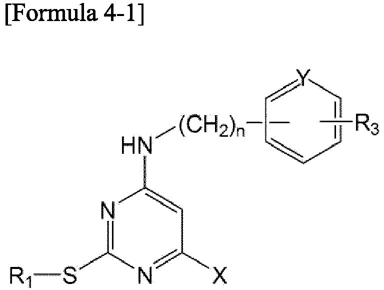

[Formula 5] R₄-X₃

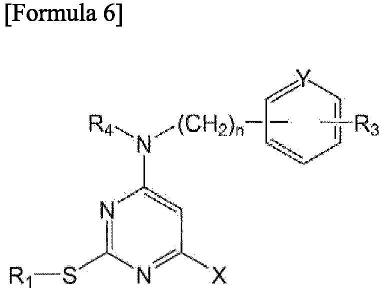

In Formulas 1, 2-1, 3, 4-1, 5 and 6,
X₁ to X₃ are each independently halogen;
X is X₁ or 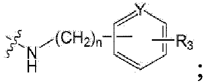
m is 0, 1, or 2;
n is an integer from 0 to 4;
Y is C(H) or N;
R₁ is C₁₋₄ alkyl;
R₂ is hydrogen, halogen, amino, -NH-(CH₂)ₚ-NHCO-R₅, or
C₆₋₁₀ aryl, C₆₋₁₀ arylamino, 3-10 membered heterocyclyl, or 3-10 membered heterocyclylamino each unsubstituted or substituted by R₆ ;
R₅ is tert-butoxy, C₆₋₁₀ aryl or 5-10 membered heteroaryl;
R₆ is amino unsubstituted or substituted by tert-butoxycarbonyl, or
tert-butoxy, C_{6-1O} aryl or 5-10 membered heteroaryl linked directly or through -CO-;
p is an integer from 1 to 4;
R₃ is hydrogen, 3-10 membered heterocyclyl, cyano, C₁₋₄ alkyl, C₁₋₄ haloalkyl, or 1 to 3 halogens; and
R₄ is hydrogen, or C₆₋₁₀ aryl-C₁₋₄ alkyl;
wherein said aryl, heteroaryl and heterocyclyl are unsubstituted or substituted by one or more selected from halogen, C₁₋₄ alkyl, C₁₋₄ alkoxy, or amino.

Step 1 may be represented by Reaction Scheme 1 below. The following reaction may be performed by the reaction with N, N-Diisopropylethylamine (DIPEA) in an organic solvent such as ethanol at 140 to 180°C for 0.5 to 5 hours but is not limited thereto. The reaction may be conducted in a microwave reactor but is not limited thereto.

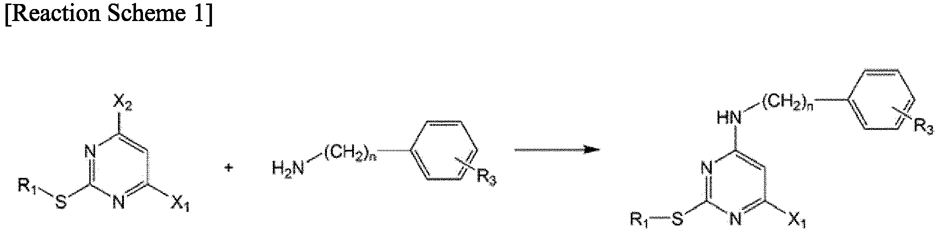

Step 2 may be represented by Reaction Scheme 2 below. The following reaction may be performed by the reaction with a base such as K₂CO₃ in the presence of tetrabutylammonium fluoride (TBAF) in an organic solvent such as acetonitrile (ACN) at 50 to 100°C for 5 to 24 hours but is not limited thereto.

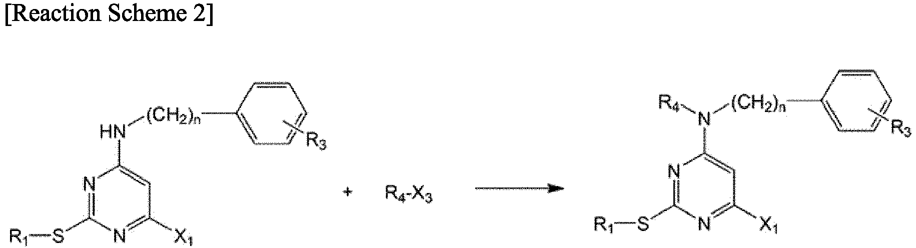

The preparation methods of the present invention may further comprise Step 3-1: preparing a compound represented by Formula 7 by reacting a compound represented by Formula 6 with ammonium hydroxide:

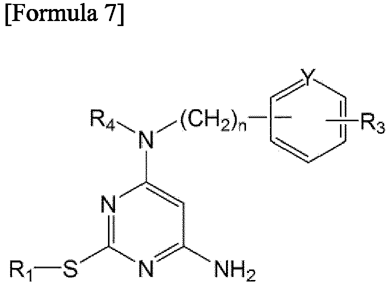

In Formula 7,
n is an integer from 0 to 4;
Yis C(H) or N;
R₁ is C₁₋₄ alkyl;
R₃ is hydrogen, 3-10 membered heterocyclyl, cyano, C₁₋₄ alkyl, C₁₋₄ haloalkyl, or 1 to 3 halogens; and
R₄ is hydrogen or C_{6-1O} aryl-C₁₋₄ alkyl;
wherein said aryl, heteroaryl and heterocyclyl are unsubstituted or substituted by one or more selected from halogen, C₁₋₄ alkyl, C₁₋₄ alkoxy, or amino.

Step 3-1 may be represented by Reaction Scheme 3 below. The following reaction may be performed by the reaction in a mixed solution of butanol and ammonium hydroxide at 140 to 180°C for 3 to 12 hours but is not limited thereto. The reaction may be conducted in a microwave reactor but is not limited thereto.

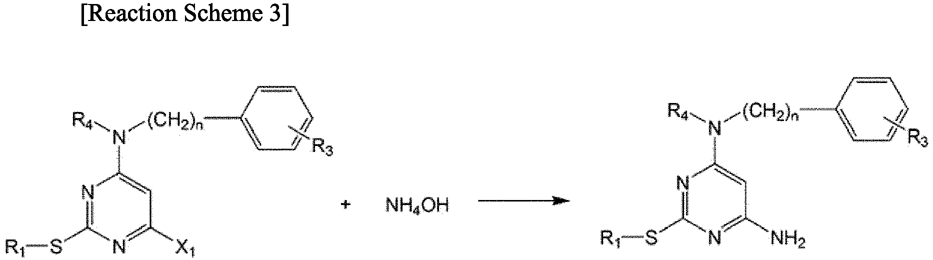

In addition, the present invention may further include the following steps :
Step 3-2: preparing a compound represented by Formulas 16 or 23, respectively, by reacting a compound represented by Formula 6 with a Boc-protected diamine derivative represented by any one of Formulas 8 to 15; and
Step 4-1: preparing a compound represented by Formulas 25 or 26, respectively, by reacting a compound represented by Formulas 16 or 17 with an acyl halide derivative represented by Formula 24, or
Step 4-2: preparing a compound represented by Formulas 27 to 33 by oxidizing the alkylthio group of the compound represented by Formulas 17 to 23 to an alkylsulfonyl group and deprotecting it.

[Formula 8] NH₂-(CH₂)ₚ-NH-Boc

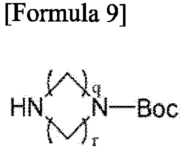

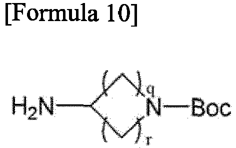

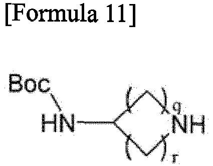

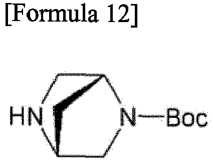

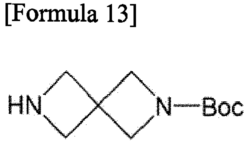

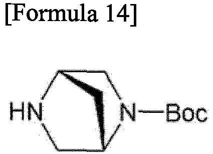

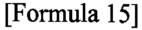

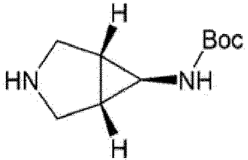

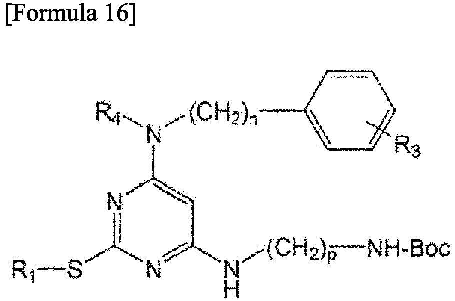

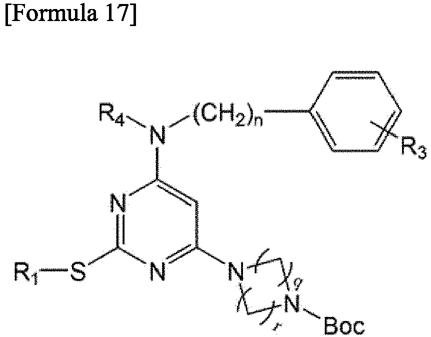

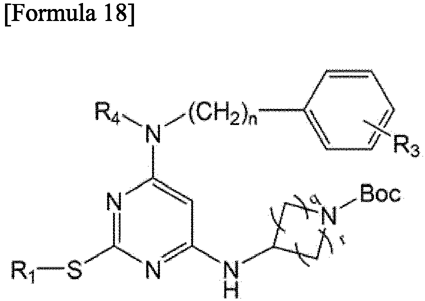

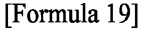

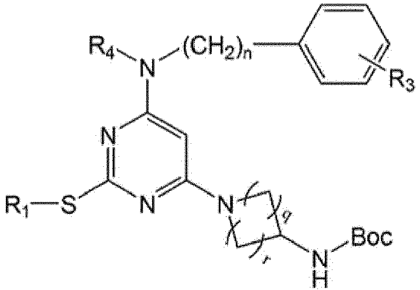

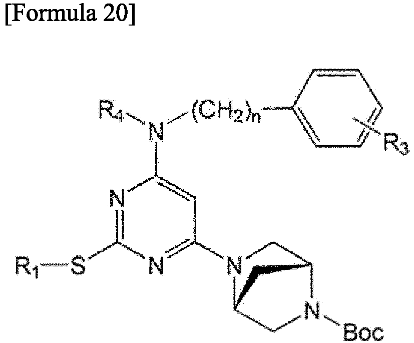

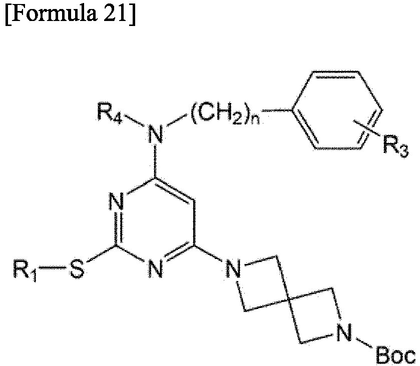

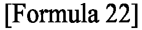

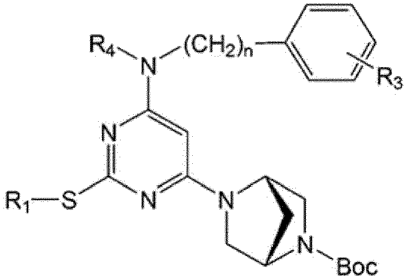

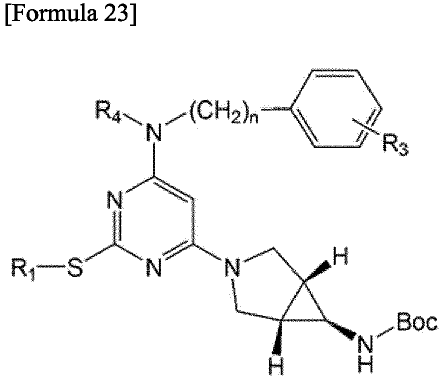

[Formula 24] R-COX₄

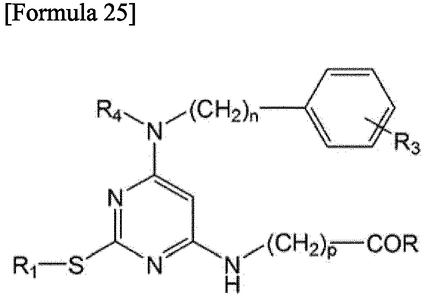

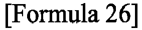

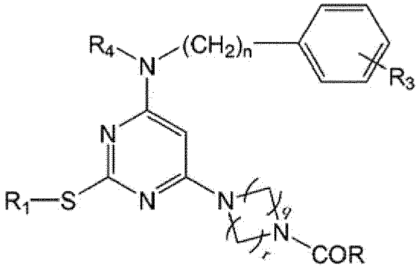

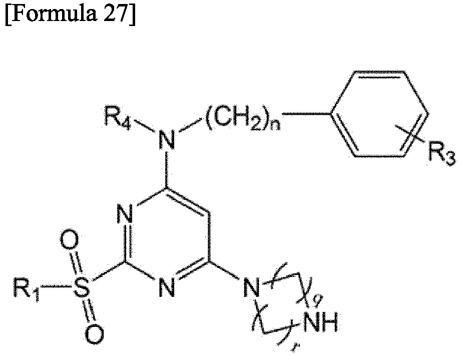

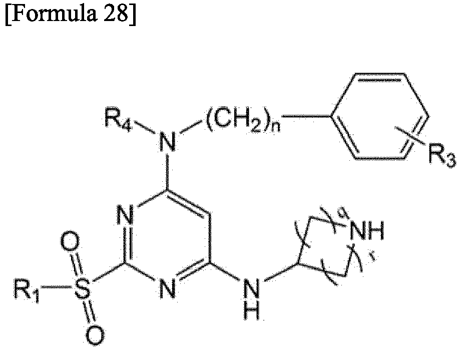

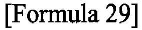

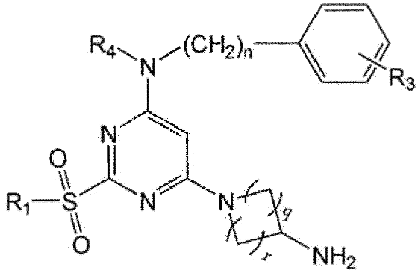

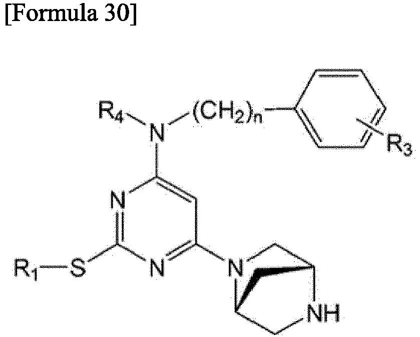

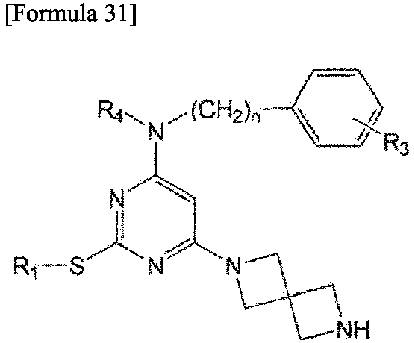

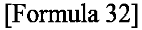

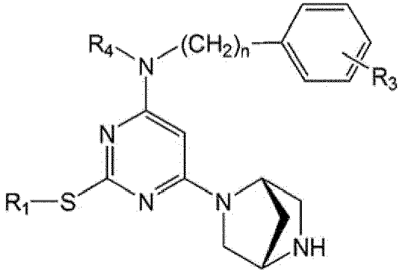

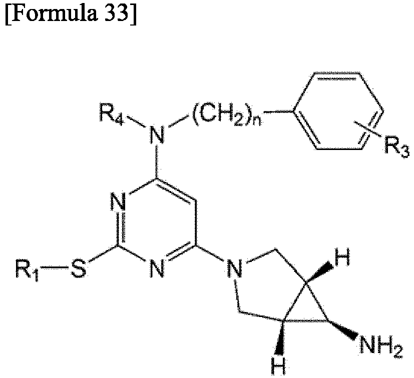

In Formulas 8 to 33,
n is an integer from 0 to 4;
p is an integer from 1 to 4;
q is 0, 1 or 2;
r is 1 or 2;
X₄ is halogen;
R₁ is C₁₋₄ alkyl;
R₃ is hydrogen, 3-10 membered heterocyclyl, cyano, C₁₋₄ alkyl, C₁₋₄ haloalkyl or 1 to 3 halogens;
R₄ is hydrogen, or C₆₋₁₀ aryl-C₁₋₄ alkyl; and
R is C_{6-1O} aryl or 5-10 membered heteroaryl;
wherein said aryl, heteroaryl and heterocyclyl are unsubstituted or substituted by one or more selected from halogen, C₁₋₄ alkyl, C₁₋₄ alkoxy or amino.

Step 3-2 may be represented by Reaction Scheme 4 below. The following reaction may be performed by the reaction with triethylamine (TEA) in an organic solvent such as tetrahydrofuran (THF) at 140 to 200°C for 0.5 to 5 hours but is not limited thereto.

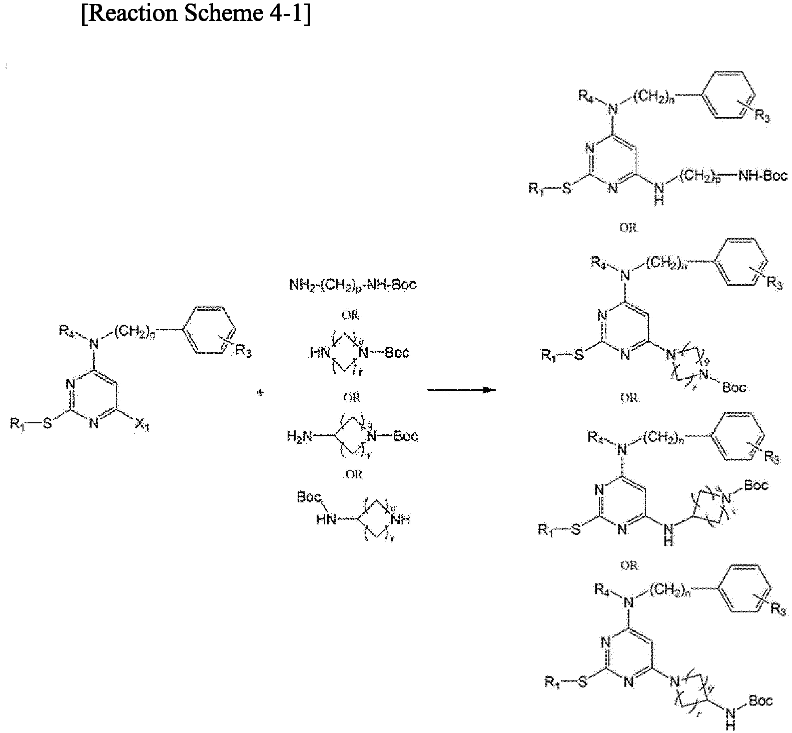

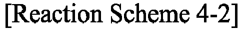

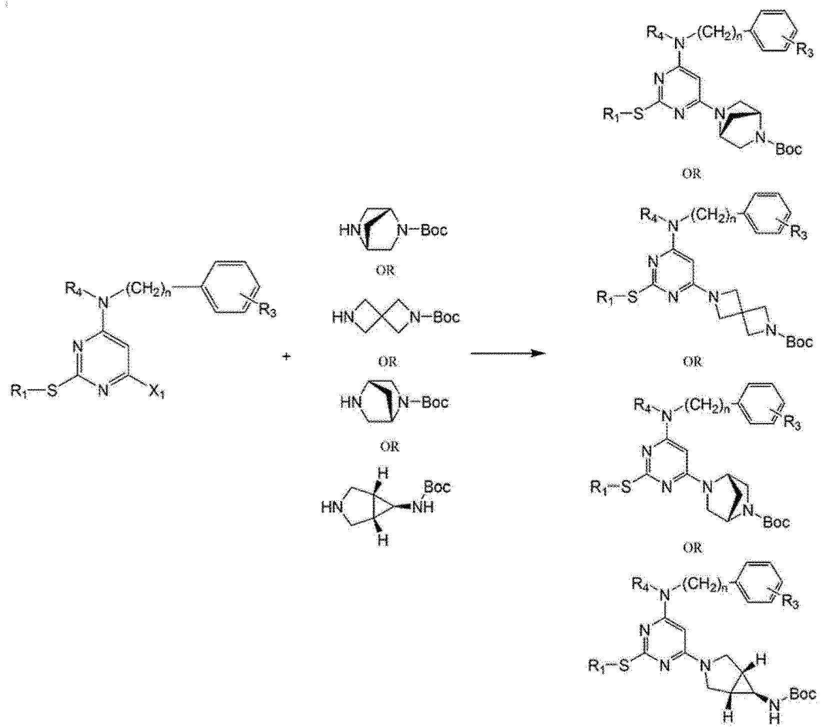

Step 4-1 may be represented by Reaction Scheme 5 below. In the following reaction, the reactant is dissolved in an organic solvent such as dichloromethane (DCM), and a DCM solution of trifluoroacetic acid (TFA) is added while stirring at a low temperature of 10°C or less and reacted for 10 minutes to 3 hours for deprotection. The obtained organic substance may be reacted with an acyl halide derivative of Formula 16 in DCM and TEA at room temperature for 0.5 to 4 hours but is not limited thereto.

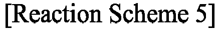

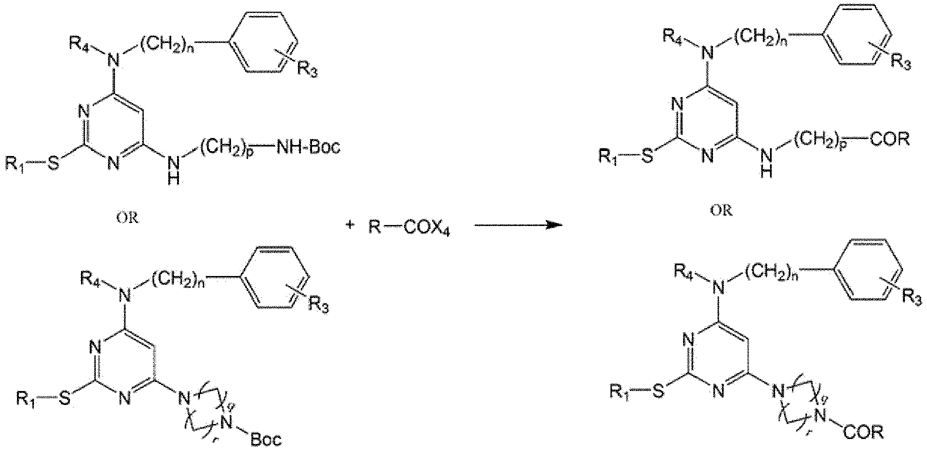

Step 4-2 may be represented by Reaction Scheme 6 below. The following reaction may be performed by mixing and reacting the reactants with mCPBA (meta-chloroperoxybenzoic acid) dissolved in an organic solvent such as DCM at room temperature for 0.5 to 4 hours but is not limited thereto. Subsequent deprotection may be performed similarly to Reaction Scheme 5 but is not limited thereto.

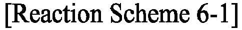

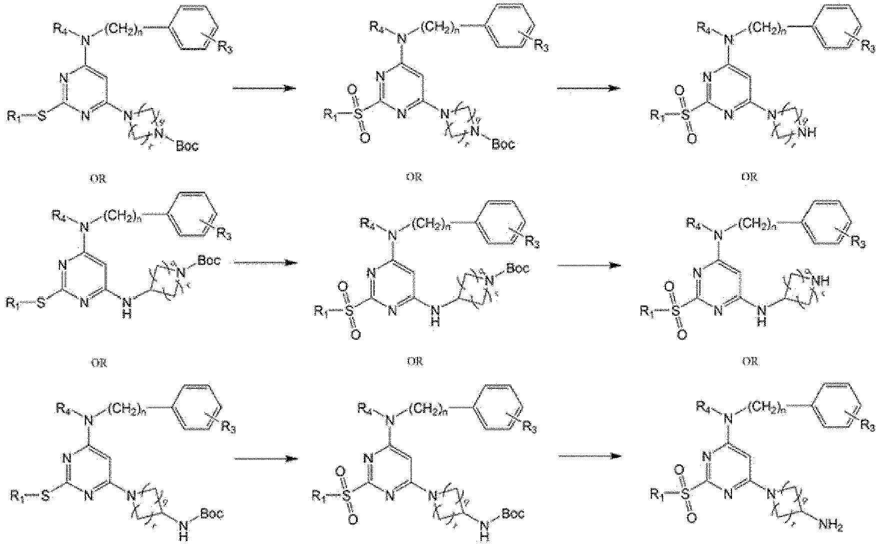

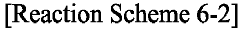

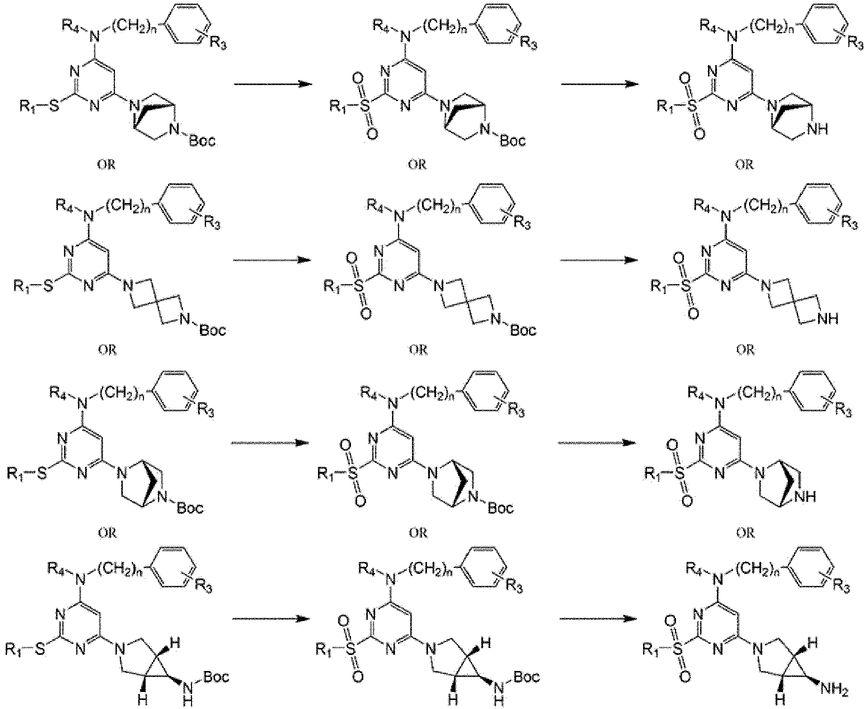

Furthermore, the preparing methods of the present invention may further comprise Step 5: oxidizing an alkylthio group to a sulfinyl group or a sulfonyl group by reacting it with mCPBA but is not limited thereto.

Step 5 may be performed similarly to Step 4-2 but is not limited thereto.

A third aspect of the present invention is a method for preparing a compound represented by Formula 1 below or a pharmaceutically acceptable salt thereof. The method comprises Step 1: preparing a compound represented by Formula 4 by reacting a compound represented by Formula 2-2 with an amine derivative represented by Formula 3; and Step 2: preparing a compound represented by Formula 34 by reacting a compound represented by Formula 4-2 with a Boc-protected diamine derivative represented by Formula 9.

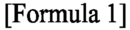

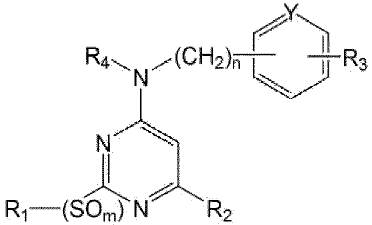

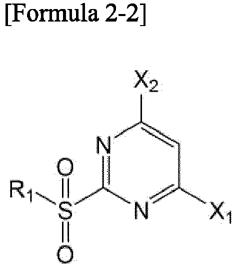

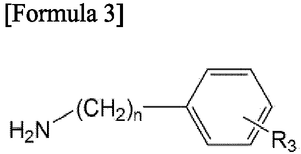

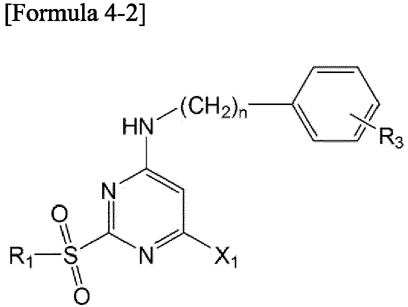

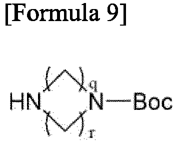

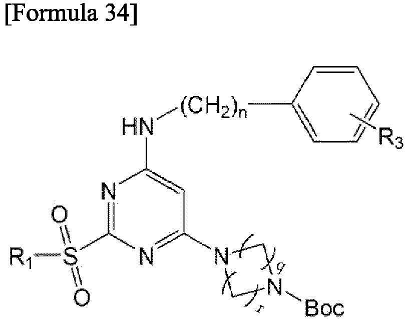

In Formulas 1, 2-2, 3, 4-2, 9 and 34,
X₁ and X₂ are each independently halogen;
m is 2;
n is an integer from 0 to 4;
Yis C(H) or N;
R₁ is C₁₋₄ alkyl;
R₂ is 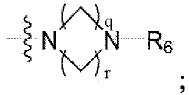
R₆ is -CO-tert-butoxy;
q is 0, 1 or 2;
r is 1 or 2;
R₃ is hydrogen, 3-10 membered heterocyclyl, cyano, C₁₋₄ alkyl, C₁₋₄ haloalkyl or 1 to 3 halogens; and
R₄ is hydrogen;
wherein said heterocyclyl is unsubstituted or substituted by one or more selected from halogen or amino.

Step 1 may be represented by Reaction Scheme 7 below. The following reaction may be performed by reacting the reactant with TEA in an organic solvent such as THF at room temperature for 10 minutes to 2 hours but is not limited thereto.

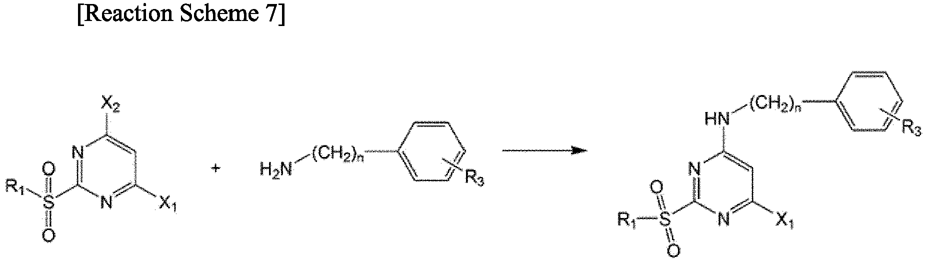

Step 2 may be represented by Reaction Scheme 8 below. The following reaction may be performed similarly to that in Reaction Scheme 4 but is not limited thereto.

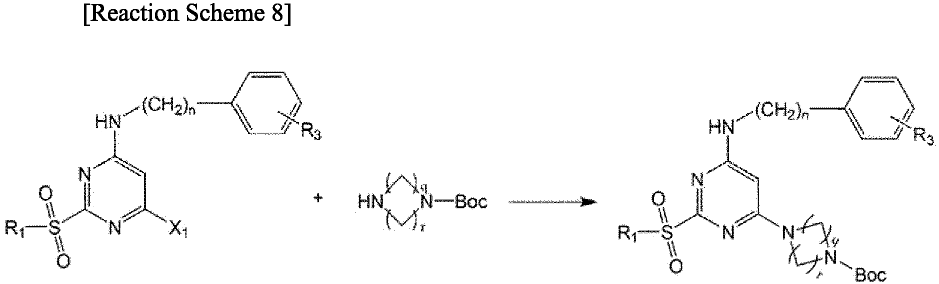

A fourth aspect of the present invention is to provide a composition for inhibiting capsid assembly, including the compound of the first aspect or a pharmaceutically acceptable salt thereof.

The terms in the present invention, "compound of the first aspect" and "pharmaceutically acceptable salt" are as described above.

The term "capsid" of the present invention means the protein shell of a virus surrounding genetic material, which is composed of subunits of several oligomeric (repetitive) structure consisting of proteins called protomers. Observable three-dimensional morphological subunits, which may or may not correspond to individual proteins, are called capsomeres. The proteins that constitute the capsid are called capsid proteins or viral coat proteins (VCP). The capsid and a genome contained therein are called nucleocapsids. The capsid is broadly classified according to the structure, and most viruses have capsids with a herical or icosahedral structure. Some viruses, such as bacteriophages, have developed into more complex structures due to constraints on elasticity and electrostatics. The capsid surface may be composed of one or more proteins. For example, a foot-and-mouth disease virus capsid has a surface composed of three proteins, VP1-3. When the virus infects a cell and start replicating itself, new capsid subunits are synthesized using the cell's protein biosynthetic mechanisms. The genetic material encapsulated by the capsid may be RNA or DNA but is not limited thereto.

For example, when a host cell is infected with a virus, it should rapidly produce thousands of identical copies of the original virus. When the virus is not inside the infected cell or is in the process of infecting the cell, the virus exists in the form of (i) genetic materials, that is, long molecules of DNA or RNA that encode the protein structures that operate the virus; (ii) a capsid, which is a protein coat that surrounds and protects the genetic material; and optionally (iii) an independent particle or virions composed of a lipid envelope.

A fifth aspect of the present invention is to provide an antiviral composition including the compound of the first aspect or a pharmaceutically acceptable salt thereof as an active ingredient.

The terms "compound of the first aspect", and "pharmaceutically acceptable salt" in the present invention are as described above.

A sixth aspect of the present invention is to provide a pharmaceutical composition for preventing or treating viral infectious diseases including the compound of the first aspect or a pharmaceutically acceptable salt thereof as an active ingredient.

The terms "compound of the first aspect", and "pharmaceutically acceptable salt" in the present invention are as described above.

The term "prevention" in the present invention means any actions that inhibit or delay the occurrence, spread and recurrence of viral infectious diseases through administration of the composition of the present invention. The term "treatment" in the present invention means any actions that improve or beneficially change the symptoms of the diseases through administration of the composition according to the present invention.

The pharmaceutical composition of the present invention may prevent or treat diseases caused by viral infection by inhibiting capsid formation.

In the pharmaceutical composition for preventing or treating viral infectious diseases, the viral infectious diseases are caused by hepatitis B virus (HBV), hepatitis C virus (HCV) or human immunodeficiency virus (HIV).

Preferably, the pharmaceutical composition according to the present invention may contain a compound represented by Formula 1, or a pharmaceutically acceptable salt thereof as an active ingredient in an amount of 0.1 to 75 % by weight, more preferably 1 to 50% by weight based on the total weight of the composition.

The composition of the present invention may further include a pharmaceutically acceptable carrier, diluent, or excipient. The composition of the present invention may be formulated and used in various forms through convention methods according to each purpose for use, such as oral formulations like powders, granules, tablets, capsules, suspensions, emulsions, syrups, aerosols, etc., and injections of sterile injection solutions. The composition of the present invention may be administered through various routes including oral administration or intravenous, intraperitoneal, subcutaneous, rectal, topical, etc. Examples of suitable carriers, excipients or diluents that may be included in such compositions may be lactose, dextrose, sucrose, sorbitol, mannitol, xylitol, erythritol, maltitol, starch, gum acacia, alginates, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate and mineral oil, etc. In addition, the composition of the present invention may further include a filler, an anti-coagulant, a lubricant, a wetting agent, a flavoring agent, an emulsifier, a preservative, etc.

Solid preparations for oral administration may include tablets, pills, powders, granules, capsules, etc. Such solid preparations may be formulated by mixing at least one or more excipients, such as starch, calcium carbonate, sucrose, lactose, gelatin, etc., with the composition. Additionally, lubricants such as magnesium stearate and talc may be used in addition to simple excipients.

Oral liquid preparations may be, for example, suspensions, internal solutions, emulsions, syrups, etc. Oral liquid preparations may include various excipients, such as wetting agents, sweeteners, aromatics, preservatives, etc., in addition to simple diluents such as water and liquid paraffin.

Preparations for parenteral administration may include sterilized aqueous solutions, non-aqueous solvents, suspensions, emulsions, freeze-drying preparations and suppositories. Propylene glycol, polyethylene glycol, vegetable oils such as olive oil and injectable esters such as ethyl oleate may be used as non-aqueous solvents and suspensions. Witepsol, Macrogol, Tween 61, cacao butter, laurin butter, glycerol gelratin, etc., may be used as a base for the suppository. Meanwhile, conventional additives such as solubilizers, isotonic agents, suspending agents, emulsifiers, stabilizers, preservatives, etc., may be included in the injection.

The composition of the present invention may be administered in a pharmaceutically effective amount. The term "pharmaceutically effective amount" of the present invention is a reasonable benefit / risk ratio applicable to medical treatment, meaning an amount that is sufficient to treat a disease and does not cause side effects. The effective dose level may be determined according to factors depending on the patient's health condition, the type of disease, severity, activity of the drug, sensitivity to the drug, method of administration, time of administration, route of administration and excretion rate, duration of treatment, drugs used in combination or concurrently and other factors well known in the medical field. The composition of the present invention may be administered as an individual therapeutic agent or in combination with other therapeutic agents. In addition, the composition of the present invention may be administered sequentially or simultaneously with conventional therapeutic agents and may be administered singly or multiple doses. Considering all of the above factors, it is important to administer an amount that can obtain the maximum effect with the minimum amount without side effects, which can be easily determined by those skilled in the art.

For example, as a dosage may increase or decrease depending on the route of administration, severity of disease, sex, weight, age, etc., the dosage does not limit the scope of the present invention in any way.

Specifically, the effective amount of the compound in the composition of the present invention may vary depending on the age, sex, and weight of the patient. In general, 1 to 100 mg per kg of body weight, preferably 5 to 60 mg, may be administered daily, every other day or divided into 1 to 3 times per day. However, as a dosage may increase or decrease depending on the route of administration, severity of disease, sex, weight, age, etc., the dosage does not limit the scope of the present invention in any way.

A fourth aspect of the present invention provides a method for treating a viral infectious disease, including a step for administering the pharmaceutical composition of the third aspect to a subject in need thereof.

The term "pharmaceutical composition of the third aspect" and "viral infectious disease" of the present invention are as described above.

The term "subject" of the present invention means any animals, including monkeys, cows, horses, sheep, pigs, chickens, turkeys, quails, cats, dogs, mice, rats, rabbits or guineas, as well as humans who have developed or may develop the viral infectious diseases. The diseases may be effectively prevented or treated by administering the pharmaceutical composition of the present invention to a subject. The pharmaceutical composition of the present invention may be administered in parallel with existing therapeutic agents.

The term "administration" of the present invention means providing a predetermined substance to a patient by any suitable method. The administration route of the composition of the present invention may be administered through any general route as long as it can reach the target tissue. The composition of the present invention may be administered by intraperitoneal administration, intravenous administration, intramuscular administration, subcutaneous administration, intradermal administration, oral administration, topical administration, intranasal administration, intrapulmonary administration or intrarectal administration, but is not limited thereto. In addition, the pharmaceutical composition of the present invention may be administered by any device capable of transporting an active substance to a target cell. Preferred administration methods and preparations may be intravenous injections, subcutaneous injections, intradermal injections, intramuscular injections, drip injections, etc. Injections may be prepared using aqueous solvents such as physiological saline, intravenous solutions, etc., or non-aqueous solvents such as vegetable oil, higher fatty acid esters (e.g., ethyl oleate, etc.), alcohols (e.g., ethanol, benzyl alcohol, propylene glycol, glycerin, etc.). Injections may include the pharmaceutical carrier such as stabilizers for preventing deterioration (e.g., ascorbic acid, sodium hydrogensulfite, sodium pyrosulfite, BHA, tocopherol, EDTA, etc.), emulsifiers, buffers to control pH, preservatives to inhibit microbial growth (e.g., phenylmercuric nitrate, thimerosal, benzalkonium chloride, phenol, cresol, benzyl alcohol, etc.).

The term "therapeutically effective amount" used in combination with an active ingredient in the present invention means an effective amount of a phenylaminopyrimidine derivative compound or a pharmaceutically acceptable salt thereof for preventing or treating a target disease.

Depending on the type of diseases to be prevented or treated, the pharmaceutical composition of the present invention may further include known drugs used for preventing or treating of each known disease as an active ingredient in addition to a phenylaminopyrimidine derivative compound, or a pharmaceutically acceptable salt thereof. For example, the composition of the present invention may further include known drugs as an active ingredient in addition to a phenylaminopyrimidine derivative compound or a pharmaceutically acceptable salt thereof when the composition is used for preventing or treating of viral infectious diseases. And using of the pharmaceutical composition of the present invention may be combined with other known treatment for treating viral infectious diseases.

### EMBODIMENTS FOR IMPLEMENTATION OF INVENTION

Hereinafter, the present invention will be described in more detail through working examples. These examples are only for explaining the present invention in more detail, and the scope of the present invention is not limited by these examples.

### Example 1: Synthetic Scheme 1

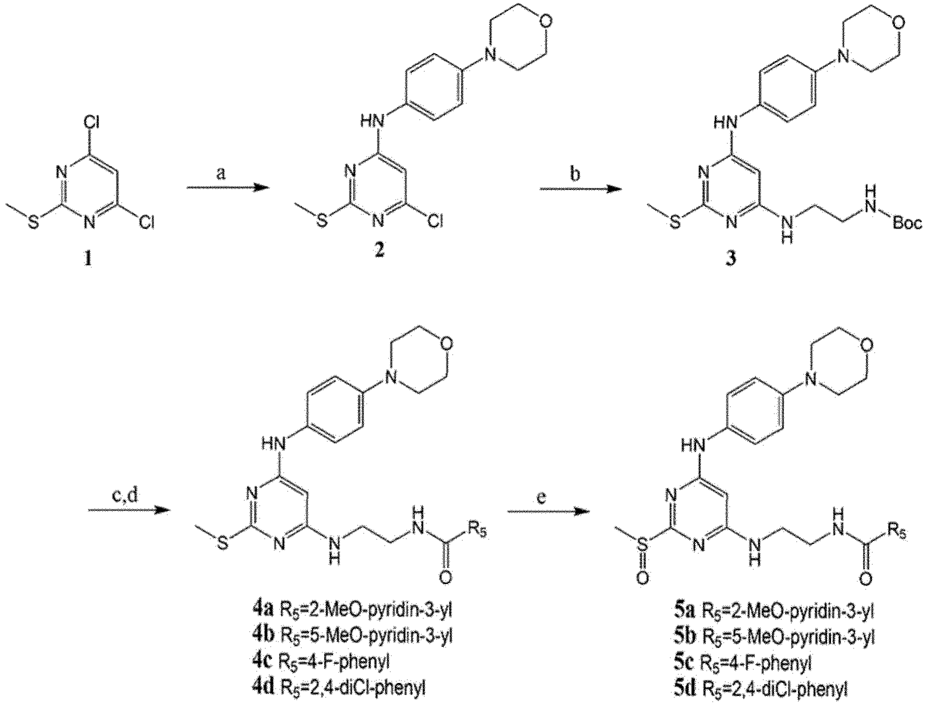

Reagents and the reaction conditions:
(a) 4-Morpholinoaniline, DIPEA, EtOH, 160°C, 3h, m.w.;
(b) N-Boc-ethylenediamine, TEA, THF, 160°C, 3h, m.w.;
(c) 20% TFA in DCM, 0°C, 1 h;
(d) R₂-COCl, TEA, DCM, rt, 2 h;
(e) mCPBA, DCM, 100°C, 0.2 h, m.w..

4,6-Dichloro-2-(methylthio)pyrimidine (Compound **1**) was mixed with 4-morpholinoaniline (2 eq.) and DIPEA (2 eq.) in ethanol (2 mL), which was reacted at 160°C for 3 hrs using a microwave reactor. After the completion of reaction was confirmed, the solution was concentrated and purified with silica-gel column chromatography to obtain Compound **2.**

Next, Compound **2** was dissolved in THF (2 mL), mixed with TEA (2 eq.) and N-Boc-ethylenediamine (2 eq.), which was reacted at 160°C for 3 hrs using a microwave reactor. After the completion of reaction was confirmed, the solution was concentrated and purified with silica-gel column chromatography to obtain Compound **3.**

Next, Compound **3** was dissolved in DCM (1 mL), and was stirred at 0 °C while slowly adding the mixed solution of TFA:DCM=1:3 (4 mL), and then was stirred for 1 hr. After the completion of reaction was confirmed, the solution was treated with sat. NaHCO₃ aqueous solution and ethyl acetate. The organic layer was extracted, dried with Na₂SO₄ and concentrated. The organic substance thus obtained was dissolved in DCM (10 mL) and TEA (2 eq.), and R₂-COCl₂ (2 eq.) corresponding to the respective R₂-group was added and reacted at r.t. for 2 hrs. After the completion of reaction was confirmed, the solution was concentrated and purified with silica-gel column chromatography to obtain Compounds **4a** to **4d.**

Lastly, each of Compounds **4a** to **4d,** respectively, was added to the solution of mCPBA (2 eq.) in DCM (2 mL), which was reacted at 100°C for 0.2 hrs using a microwave reactor. The solution was treated using NaHCO₃ aqueous solution and DCM. The organic layer was extracted, dried with Na₂SO₄ and concentrated. The concentrated organic substance was purified with silica-gel column chromatography to obtain Compounds **5a** to **5d.**

### Example 2: Synthetic Scheme 2

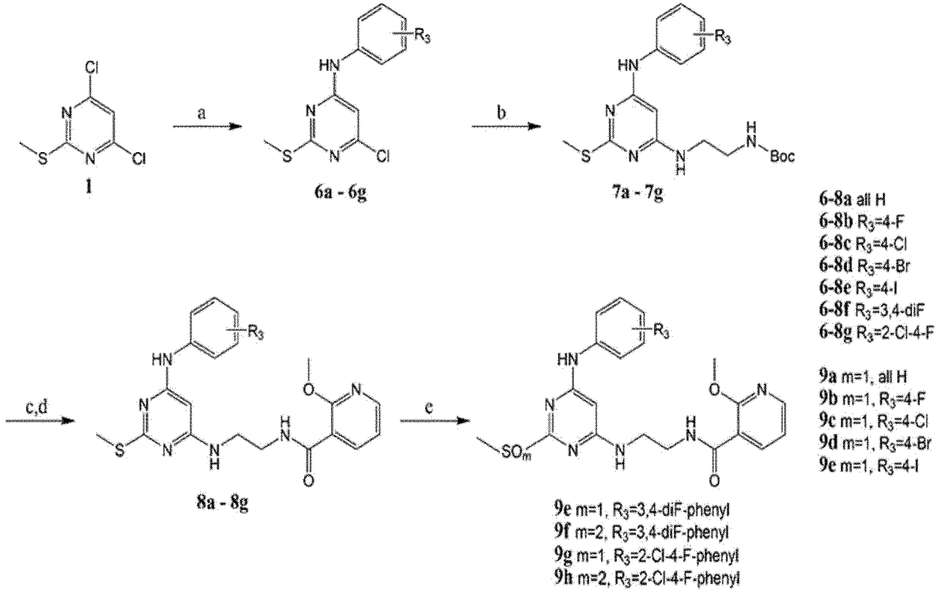

Reagents and the reaction conditions:
(a) R₃-aniline, DIPEA, EtOH, m.w., 160°C, 3h;
(b) N-Boc-ethylenediamine, TEA, THF, 180°C, 3h;
(c) 20% TFA in DCM, 0°C, 1 h;
(d) 2-methoxynicotinoyl chloride, TEA, DCM, r.t., 2 h;
(e) mCPBA, MeOH, r.t., 0.5 h.

Compound 1 was mixed with R₃-aniline (2 eq.) corresponding to the respective R₃-group and DIPEA (2 eq.) in EtOH (2 mL), which was reacted at 160°C for 3 hrs using a microwave reactor. After the completion of reaction was confirmed, the solution was concentrated and purified with silica-gel column chromatography to obtain Compounds **6a** to **6g.**

Next, each of Compounds **6a** to **6g,** respectively, was dissolved in THF (2 mL), mixed with TEA (2 eq.) and N-Boc-ethylenediamine (2 eq.), and reacted at 160°C for 3 hrs using a microwave reactor. After the completion of reaction was confirmed, the solution was concentrated and purified with silica-gel column chromatography to obtain Compounds **7a** to **7g.**

Next, each of Compounds **7a** to **7g,** respectively, was dissolved in DCM (1 mL), stirred at 0 °C while slowly adding the mixed solution of TFA:DCM=1:3 (4 mL), and then was stirred for 1 hr. After the completion of reaction was confirmed, the solution was concentrated and treated with sat. NaHCO₃ aqueous solution and ethyl acetate. The organic layer was extracted, dried with Na₂SO₄ and concentrated. The organic substance thus obtained was dissolved in DCM (10 mL) and TEA (2 eq.), and 2-methoxynicotinoyl chloride (2 eq.) was added and reacted at r.t. for 2 hrs. After the completion of reaction was confirmed, the solution was concentrated and purified with silica-gel column chromatography to obtain Compounds **8a** to **8g.**

Lastly, each of Compounds **8a** to **8g,** respectively, was added to the solution of mCPBA (2 eq.) in DCM (2 mL), which was reacted at 100°C for 0.2 hrs using a microwave reactor. The solution was treated using NaHCO₃ aqueous solution and DCM. The organic layer was extracted, dried with Na₂SO₄ and concentrated. The concentrated organic substance was purified with silica-gel column chromatography to obtain Compounds **9a** to **9g.**

### Example 3: Synthetic Scheme 3

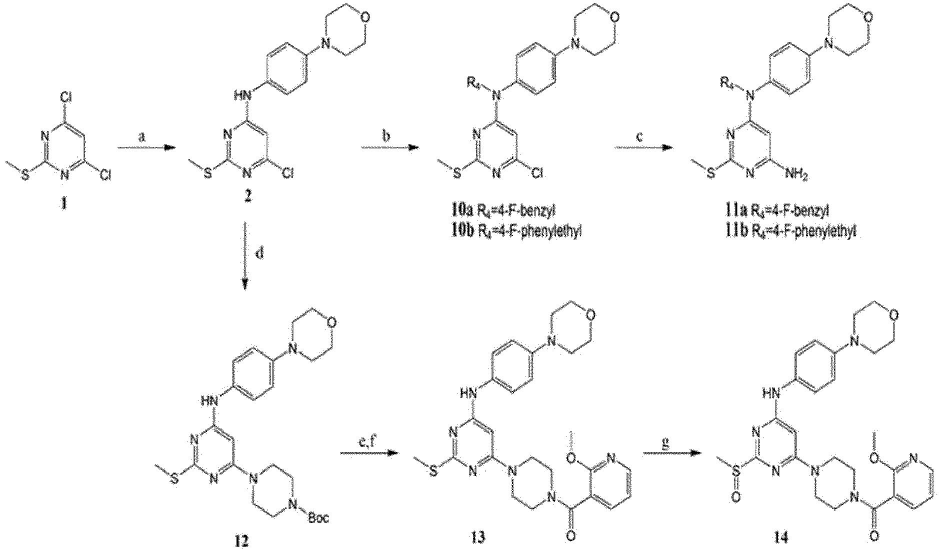

Reagents and the reaction conditions:
(a) 4-Morpholinoaniline, DIPEA, EtOH, 160°C, 3h, m.w.;
(b) R₄-Br, K₂CO₃, TBAF, ACN, 70°C, 12h;
(c) NH₄OH (20% aq.), n-BuOH, m.w., 160°C, 6h;
(d) N-Boc-piperazine, TEA, THF, m.w., 160°C, 3h;
(e) 20% TFA in DCM, 0°C, 1 h;
(f) 2-methoxynicotinoyl chloride, TEA, THF, r.t., 1 h;
(g) mCPBA, DCM, 100°C, 0.2 h, m.w..

Compound 1 was mixed with 4-morpholinoaniline (2 eq.) and DIPEA (2 eq.) in EtOH (2 mL), which was reacted at 160°C for 3 hrs using a microwave reactor. After the completion of reaction was confirmed, the solution was concentrated and purified with silica-gel column chromatography to obtain Compound **2.**

Next, Compound **2** was dissolved in ACN (10 mL), and K₂CO₃ (2eq.), TBAF (0.05 eq.) and R₄-BR (2 eq.) corresponding the respective R₄-group were dissolved altogether. The mixture was reacted at 70°C for 12 hrs. After the completion of reaction was confirmed, the solution was concentrated and purified with silica-gel column chromatography to obtain Compounds **10a** and **10b.**

Next, each of Compounds **10a** and **10b,** respectively, was mixed together with 1 mL of mixed solution of NH₄OH (20% aq.):n-BuOH = 1:1, which was reacted at 160°C for 6 hrs using a microwave reactor. After the completion of reaction was confirmed, the solution was treated with sat. NaHCO₃ aqueous solution and ethyl acetate. The organic layer was extracted, dried with Na₂SO₄, concentrated, and purified with silica-gel column chromatography to obtain Compounds **11a** and **11b.**

Separately, Compound **2** was dissolved in THF (2 mL) and mixed with TEA (2 eq.) and N-Boc-piperazine (2 eq.), which was reacted at 160°C for 3 hrs using a microwave reactor. After the completion of reaction was confirmed, the solution was concentrated and purified with silica-gel column chromatography to obtain Compound **12.**

Next, Compound **12** was dissolved in DCM (1 mL), stirred at 0 °C while slowly adding the mixed solution of TFA:DCM=1:3 (4 mL), and then was stirred for 1 hr. After the completion of reaction was confirmed, the solution was concentrated and treated with sat. NaHCO₃ aqueous solution and ethyl acetate. The organic layer was extracted, dried with Na₂SO₄ and concentrated. The organic substance thus obtained was dissolved in DCM (10 mL) and TEA (2 eq.), and 2-methoxynicotinoyl chloride (2 eq.) was added and reacted at r.t. for 2 hrs. After the completion of reaction was confirmed, the solution was concentrated and purified with silica-gel column chromatography to obtain Compound **13.**

Lastly, Compound **13** was added to the solution of mCPBA (2 eq.) in DCM (2 mL), which was reacted at 100°C for 0.2 hrs using a microwave reactor. The solution was treated using NaHCO₃ aqueous solution and DCM. The organic layer was extracted, dried with Na₂SO₄ and concentrated. The concentrated organic substance was purified with silica-gel column chromatography to obtain Compound **14.**

### Example 4: Synthetic Scheme 4

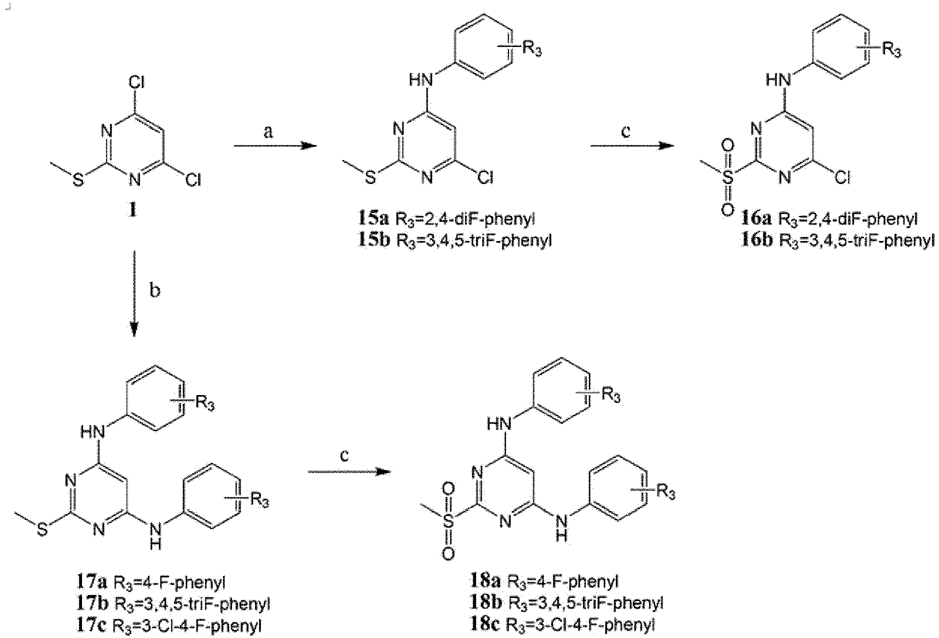

Reagents and the reaction conditions:
(a) R₃-aniline, DIPEA, EtOH, m.w., 160°C, 3h;
(b) R₃-aniline, CuI (I), H2O, 160°C, m.w., 1.5h;
(c) mCPBA, MeOH, r.t., 0.5h.

Compound **1** was mixed with R₃-aniline (2 eq.) corresponding to the respective R₃-group and DIPEA (2 eq.) in EtOH (2 mL), which was reacted at 160°C for 3 hrs using a microwave reactor. After the completion of reaction was confirmed, the solution was concentrated and purified with silica-gel column chromatography to obtain Compounds **15a** and **15b.**

Separately, Compound **1** was mixed with water (1 mL), CuI (I) (0.05 eq.) and R₃-aniline (2 eq.) corresponding to the respective R₃-group, which was reacted at 160°C for 1.5 hrs using a microwave reactor. After the completion of reaction was confirmed, the solution was treated with sat. NaHCO₃ aqueous solution and ethyl acetate. The organic layer was extracted, dried with Na₂SO₄, concentrated, and purified with silica-gel column chromatography to obtain Compounds **17a** to **17c.**

Lastly, each of Compounds **15a, 15b,** and **17a** to **17c,** respectively, was added to the solution of mCPBA (2 eq.) in DCM (2 mL), which was reacted at 100°C for 0.2 hrs using a microwave reactor. The solution was treated using NaHCO₃ aqueous solution and DCM. The organic layer was extracted, dried with Na₂SO₄ and concentrated. The concentrated organic substance was purified with silica-gel column chromatography to obtain Compounds **16a, 16b,** and **18a** to **18c.**

### Example 5: Synthetic Scheme 5

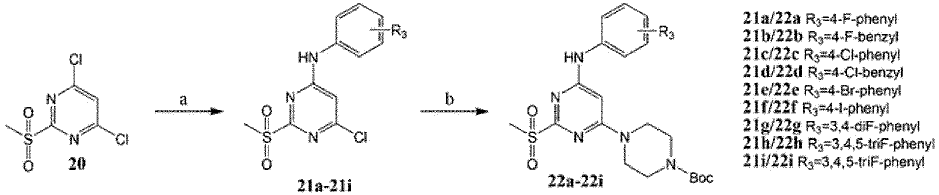

Reagents and the reaction conditions:
(a) R₃-aniline, TEA, THF, r.t., 1h;
(b) N-Boc-piperazine, TEA, THF, 160°C, m.w., 3h.

4,6-dichloro-2-(methylsulfonyl)pyrimidine (Compound **20)** was mixed with R₃-aniline (2 eq.) corresponding to the respective R₃-group and TEA (2 eq.) in THF (2 mL) and was reacted at r.t. for 1 hr. After the completion of reaction was confirmed, the solution was concentrated and purified with silica-gel column chromatography to obtain Compounds **21a** to **21i.**

Next, each of Compounds **21a** to **21i,** respectively, was dissolved in THF (2 mL), mixed with TEA (2 eq.) and N-Boc-piperazine (2 eq.), and reacted at 160°C for 3 hrs using a microwave reactor. After the completion of reaction was confirmed, the solution was concentrated and purified with silica-gel column chromatography to obtain Compounds **22a** to **22i.**

### Example 6: Synthetic Scheme 6

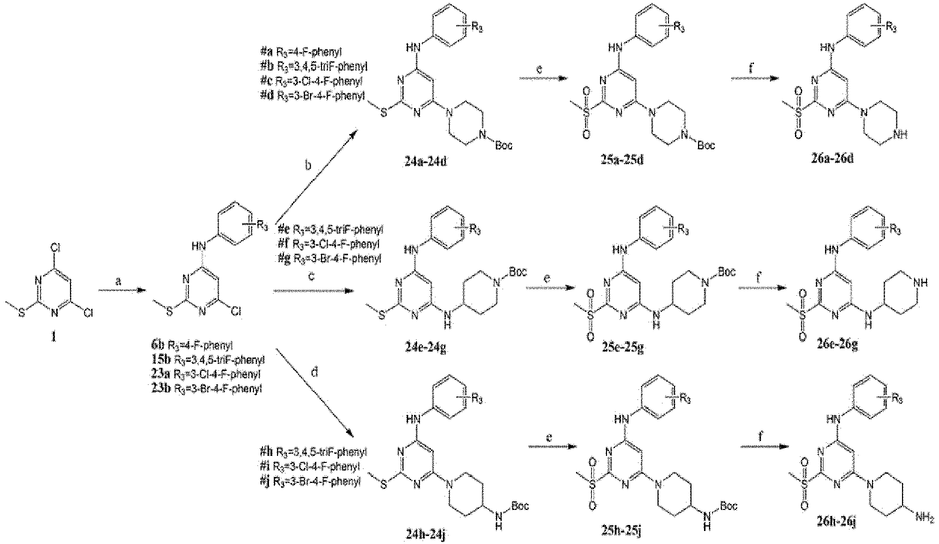

Reagents and the reaction conditions:
(a) R₃-aniline, DIPEA, EtOH, m.w., 160°C, 3h;
(b) N-Boc-piperazine, TEA, THF, m.w., 160°C, 3h;
(c) 4-amino-1-Boc-piperidine, TEA, THF, m.w., 160°C, 3h;
(d) 4-(N-Boc-amino)piperidine, TEA, THF, m.w., 160°C, 3h;
(e) mCPBA, DMF. R.t., 2h;
(f) 20% TFA in DCM, 0°C, 1 h.

Compound **1** was mixed with R₃-aniline (2 eq.) corresponding to the respective R₃-group and DIPEA (2 eq.) in EtOH (2 mL), which was reacted at 160°C for 3 hrs using a microwave reactor. After the completion of reaction was confirmed, the solution was concentrated and purified with silica-gel column chromatography to obtain Compound **6b,** Compound **15b,** Compound **23a,** and Compound **23b.**

Next, each of Compound **6b,** Compound **15b,** Compound **23a,** and Compound **23b,** respectively, was dissolved in THF (2 mL), mixed with TEA (2 eq.) and N-Boc-piperazine (2 eq.), and reacted at 160°C for 3 hrs using a microwave reactor. After the completion of reaction was confirmed, the solution was concentrated and purified with silica-gel column chromatography to obtain Compounds **24a** to **24d.**

In addition, by following the similar reaction pathways as above, except that each of Compound **15b,** Compound **23a,** and Compound **23b,** respectively, was reacted with 4-amino-1-Boc-piperidine (2 eq.) instead of N-Boc-piperazine, Compounds **24e** to **24g** were obtained.

Further, by following the similar reaction pathways as above, except that each of Compound **15b,** Compound **23a,** and Compound **23b,** respectively, was reacted with 4-(N-Boc-amino)piperidine (2 eq.) instead of N-Boc-piperazine, Compounds **24h** to **24j** were obtained.

Next, each of Compounds **24a to 24j,** respectively, was added to the solution of mCPBA (2 eq.) in DCF (2 mL), which was reacted at r.t. for 2 hrs. The organic solvent was removed by vacuum pump, and the solution was treated using sat. NaHCO₃ aqueous solution and DCM. The organic layer was extracted, dried with Na₂SO₄ and concentrated. The concentrated organic substance was purified with silica-gel column chromatography to obtain Compounds **25a** to **25j.**

Lastly, each of Compounds **25a** to **25j,** respectively, was dissolved in DCM (1 mL), stirred at 0 °C while slowly adding the mixed solution of TFA:DCM=1:3 (4 mL), and then was stirred for 1 hr. After the completion of reaction was confirmed, the solution was concentrated and treated with sat. NaHCO₃ aqueous solution and ethyl acetate. The organic layer was extracted, dried with Na₂SO₄ and concentrated. The concentrated organic substance was purified with silica-gel column chromatography to obtain Compounds **26a to 26j.**

### Example 7: Synthetic Scheme 7

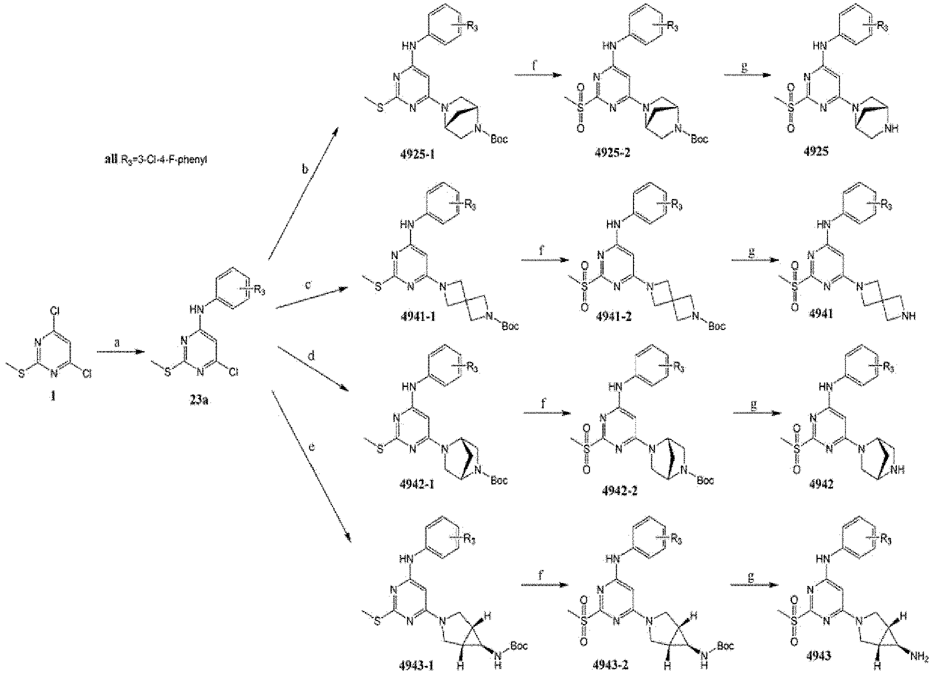

(a) 3-Chloro-4-fluoroaniline(R₃-aniline), DIPEA, EtOH, m.w., 160°C, 3h;
(b) (1R, 4R)-tert-butyl 2,5-diazabicyclo[2.2.1]heptan-2-carboxylate, TEA, THF, m.w., 160°C, 3h;
(c) Tert-butyl 2,6-diazaspiro[3.3]heptan-2-carboxylate, TEA, THF, m.w., 160°C, 3h;
(d) (1s, 4s)-tert-butyl 2,5-diazabicyclo[2.2.1]heptan-2-carboxylate, TEA, THF, m.w., 160°C, 3h;
(e) exo-6-(boc-amino)-3-azabicyclo[3.1.0]hexane, TEA, THF, m.w., 160°C, 3h;
(f) mCPBA, DMF, r.t., 2h;
(g) 20% TFA in DCM, 0°C, 1h.

Compound 1 was mixed with 3-chloro-4-fluoroaniline (2 eq.) and DIPEA (2 eq.) in EtOH (2 mL), which was reacted at 160°C for 3 hrs using a microwave reactor. After the completion of reaction was confirmed, the solution was concentrated and purified with silica-gel column chromatography to obtain Compound **23a.**

Next, Compound **23a** was dissolved in THF (2 mL), mixed with TEA (2 eq.) and (1R, 4R)-tert-butyl 2,5-diazabicyclo[2.2.1]heptan-2-carboxylate (2 eq.), and reacted at 160°C for 3 hrs using a microwave reactor. After the completion of reaction was confirmed, the solution was concentrated and purified with silica-gel column chromatography to obtain Compound **4925-1.**

In addition, by following the similar reaction pathways as above, except that Compound **23a** was reacted with tert-butyl 2,6-diazaspiro[3.3]heptan-2-carboxylate (2 eq.) instead of (1R, 4R)-tert-butyl 2,5-diazabicyclo[2.2.1]heptan-2-carboxylate, Compound **4941-1** was obtained.

Further, by following the similar reaction pathways as above, except that Compound **23a** was reacted with (1s, 4s)-tert-butyl 2,5-diazabicyclo[2.2.1]heptan-2-carboxylate (2 eq.) instead of (1R, 4R)-tert-butyl 2,5-diazabicyclo[2.2.1]heptan-2-carboxylate, Compound **4942-1** was obtained.

Further, by following the similar reaction pathways as above, except that Compound **23a** was reacted with exo-6-(boc-amino)-3-azabicyclo[3.1.0]hexane (2 eq.) instead of (1R, 4R)-tert-butyl 2,5-diazabicyclo[2.2.1]heptan-2-carboxylate, Compound **4943-1** was obtained.

Next, each of Compound **4925-1,** Compound **4941-1,** Compound **4942-1,** and Compound **4943-1,** respectively, was added to the solution of mCPBA (2 eq.) in DCF (2 mL), which was reacted at r.t. for 2 hrs. The organic solvent was removed by vacuum pump, and the solution was treated using sat. NaHCO₃ aqueous solution and DCM. The organic layer was extracted, dried with Na₂SO₄ and concentrated. The concentrated organic substance was purified with silica-gel column chromatography to obtain Compound **4925-2,** Compound **4941-2,** Compound **4942-2,** and Compound **4943-2,** respectively.

Lastly, each of Compound **4925-2,** Compound **4941-2,** Compound **4942-2,** and Compound **4943-2,** respectively, was dissolved in DCM (1 mL), stirred at 0 °C while slowly adding the mixed solution of TFA:DCM=1:3 (4 mL), and then was stirred for 1 hr. After the completion of reaction was confirmed, the solution was concentrated and treated with sat. NaHCO₃ aqueous solution and ethyl acetate. The organic layer was extracted, dried with Na₂SO₄ and concentrated. The concentrated organic substance was purified with silica-gel column chromatography to obtain Compound **4925,** Compound **4941,** Compound **4942,** and Compound **4943,** respectively.

### Example 8 : Identification of novel compounds

A series of compounds synthesized according to Examples 1 to 7 was identified with ¹H NMR and mass spectrometry, and the results were shown below along with the compound names. The numbers of the compounds below correspond to the numbers indicated in the scheme above.

### Compound 4a: 2-methoxy-N-(2-(2-(methylthio)-6-(4-morpholinophenylamino)pyrimidin-4-ylamino)ethyl)nicotinamide (LDD-3139)

¹H NMR (CDCl₃, 400 MHz): δ 7.53 (d, *J* = 8.2 Hz, 1 H), 7.41 (d, *J* = 1.8 Hz, 1 H), 7.30 (dd, *J* = 8.4, 2.0 Hz, 2 H), 7.15 (d, *J* = 8.5 Hz, 2 H), 6.93 (d, *J* = 8.9 Hz, 2 H), 6.68 (s, 1 H), 5.52 (s, 1 H), 3.86 - 3.91 (m, 5 H), 3.63 - 3.68 (m, 2 H), 3.58 (br. s., 2 H), 3.16 - 3.20 (m, 4 H), 2.82 ppm (s, 3 H);
MS (ESI): [M + H]⁺ = 495.99.

### Compound 4b: 5-methoxy-N-(2-(2-(methylthio)-6-(4-morpholinophenylamino)pyrimidin-4-ylamino)ethyl)nicotinamide (LDD-3345)

¹H NMR (CDCl₃, 400 MHz): δ 8.55 (d, *J* = 1.5 Hz, 1 H), 8.42 (d, *J* = 3.1 Hz, 1 H), 7.61 (dd, *J* = 2.7, 1.8 Hz, 1 H), 7.12 - 7.17 (m, *J* = 8.9 Hz, 2 H), 6.88 - 6.92 (m, *J* = 8.9 Hz, 2 H), 6.34 (s, 1 H), 5.30 (s, 1 H), 4.88 (br. s., 1 H), 3.90 (s, 3 H), 3.86 - 3.89 (m, 4 H), 3.63 (br. s., 5 H), 3.14 - 3.17 (m, 4 H), 2.48 ppm (s, 3 H);
MS (ESI): [M + H]⁺ = 495.92.

### Compound 4c: 4-fluoro-N-(2-(2-(methylthio)-6-(4-morpholinophenylamino)pyrimidin-4-ylamino)ethyl)benzamide (LDD-3157)

¹H NMR (CDCl₃, 400 MHz): δ 8.13 (dd, *J* = 8.8, 5.3 Hz, 1 H), 7.83 (dd, *J* = 8.8, 5.3 Hz, 1 H), 7.10 - 7. (m, 3 H), 7.07 (t, *J* = 8.6 Hz, 1 H), 5.28 (d, *J* = 3.8 Hz, 1 H), 2.50 - 2.53 ppm (m, 3 H);
MS (ESI): [M + H]⁺ = 482.97.

### Compound 4d: 2,4-dichloro-N-(2-(2-(methylthio)-6-(4-morpholinophenylamino)pyrimidin-4-ylamino)ethyl)benzamide (LDD-3155)

¹H NMR (CDCl₃, 400 MHz): δ 7.48 (d, *J* = 8.2 Hz, 1 H), 7.39 (d, *J* = 2.1 Hz, 1 H), 7.28 (d, *J* = 2.1 Hz, 1 H), 7.14 (d, *J* = 8.9 Hz, 3 H), 6.90 (d, *J* = 8.9 Hz, 2 H), 6.47 (s, 1 H), 5.29 (s, 1 H), 3.84 - 3.89 (m, 5 H), 3.59 - 3.64 (m, 2 H), 3.56 (br. s., 2 H), 3.12 - 3.17 ppm (m, 5 H);
MS (ESI): [M - H]⁻ = 532.64.

### Compound 5a: 2-methoxy-N-(2-(2-(methylsulfinyl)-6-(4-morpholinophenylamino)pyrimidin-4-ylamino)ethyl)nicotinamide (LDD-3140)

¹H NMR (CDCl₃, 400 MHz: δ 8.47 (dd, *J* = 7.6, 2.3 Hz, 1 H), 8.29 (dd, *J* = 4.8, 2.1 Hz, 1 H), 8.18 (t, *J* = 6.3 Hz, 1 H), 7.11 - 7.17 (m, *J* = 8.4 Hz, 2 H), 7.06 (dd, *J* = 7.4, 4.8 Hz, 1 H), 6.89 - 6.94 (m, *J* = 8.0 Hz, 2 H), 6.74 (br. s., 1 H), 5.60 (t, *J* = 4.2 Hz, 1 H), 5.55 (s, 1 H), 4.07 (s, 3 H), 3.86 - 3.91 (m, 4 H), 3.67 (q, *J* = 5.8 Hz, 2 H), 3.51 (br. s., 2 H), 3.17 (br. s., 4 H), 2.87 ppm (s, 3 H);
MS (ESI): [M + H]⁺ = 512.04.

### Compound 5b: 5-methoxy-N-(2-(2-(methylsulfinyl)-6-(4-morpholinophenylamino)pyrimidin-4-ylamino)ethyl)nicotinamide (LDD-3366)

¹H NMR (CDCl₃, 400 MHz): δ 8.77 (s, 1 H), 8.36 (d, *J* = 2.7 Hz, 1 H), 7.82 (br. s., 1 H), 7.69 - 7.71 (m, 1 H), 7.11 - 7.15 (m, 2 H), 6.89 - 6.92 (m, *J* = 8.9 Hz, 2 H), 5.51 (s, 1 H), 3.87 - 3.90 (m, 7 H), 3.63 - 3.71 (m, 2 H), 3.53 (br. s., 2 H), 3.14 - 3.19 (m, 4 H), 2.86 ppm (s, 3 H);
MS (ESI): [M + H]⁺ = 512.13.

### Compound 5c: 4-fluoro-N-(2-(2-(methylsulfinyl)-6-(4-morpholinophenylamino)pyrimidin-4-ylamino)ethyl)benzamide (LDD-3160)

¹H NMR (CDCl₃, 400 MHz): δ 8.77 (s, 1 H), 8.36 (d, *J* = 2.7 Hz, 1 H), 7.82 (br. s., 1 H), 7.69 - 7.71 (m, 1 H), 7.11 - 7.15 (m, 2 H), 6.89 - 6.92 (m, *J* = 8.9 Hz, 2 H), 5.51 (s, 1 H), 3.87 - 3.90 (m, 7 H), 3.63 - 3.71 (m, 2 H), 3.53 (br. s., 2 H), 3.14 - 3.19 (m, 4 H), 2.86 ppm (s, 3 H);
MS (ESI): [M + H]⁺ = 512.13.

### Compound 5d: 2,4-dichloro-N-(2-(2-(methylsulfinyl)-6-(4-morpholinophenylamino)pyrimidin-4-ylamino)ethyl)benzamide (LDD-3156)

¹H NMR (CDCl₃, 400 MHz): δ 7.53 (d, *J* = 8.2 Hz, 1 H), 7.41 (d, *J* = 1.8 Hz, 1 H), 7.30 (dd, *J* = 8.4, 2.0 Hz, 2 H), 7.15 (d, *J* = 8.5 Hz, 2 H), 6.93 (d, *J* = 8.9 Hz, 2 H), 6.68 (s, 1 H), 5.52 (s, 1 H), 3.86 - 3.91 (m, 5 H), 3.63 - 3.68 (m, 2 H), 3.58 (br. s., 2 H), 3.16 - 3.20 (m, 4 H), 2.82 ppm (s, 3 H);
MS (ESI): [M + H]⁺ = 548.19.

### Compound 8a: 2-methoxy-N-(2-(2-(methylthio)-6-(phenylamino)pyrimidin-4-ylamino)ethyl)nicotinamide (LDD-3343)

¹H NMR (CDCl₃, 400 MHz): δ 8.48 (dd, *J* = 7.6, 2.1 Hz, 1 H), 8.27 (dd, *J* = 4.9, 2.1 Hz, 1 H), 8.15 (t, *J* = 6.0 Hz, 1 H), 7.31 - 7.37 (m, 2 H), 7.22 - 7.26 (m, 2 H), 7.09 - 7.14 (m, 1 H), 7.05 (dd, *J* = 7.6, 4.9 Hz, 1 H), 6.47 (s, 1 H), 5.55 (s, 1 H), 5.13 (t, *J* = 5.3 Hz, 1 H), 4.05 (s, 3 H), 3.64 - 3.71 (m, 2 H), 3.53 - 3.62 ppm (m, 2 H);
[M - H]⁻ = 408.95.

### Compound 8b: N-(2-(6-(4-fluorophenylamino)-2-(methylthio)pyrimidin-4-ylamino)ethyl)-2-methoxynicotinamide (LDD-3346)

¹H NMR (CDCl₃, 400 MHz): δ 8.44 - 8.51 (m, 1 H), 8.25 - 8.30 (m, 1 H), 8.15 (t, *J* = 4.7 Hz, 1 H), 7.18 - 7.25 (m, 2 H), 6.99 - 7.10 (m, 3 H), 6.36 (s, 1 H), 5.39 (s, 1 H), 5.13 (t, *J* = 5.6 Hz, 1 H), 4.05 (s, 3 H), 3.67 (q, *J=* 5.9 Hz, 2 H), 3.50 - 3.61 (m, 2 H), 2.49 ppm (s, 3 H);
[M - H]⁻ = 226.73.

### Compound 8c: N-(2-(6-(4-chlorophenylamino)-2-(methylthio)pyrimidin-4-ylamino)ethyl)-2-methoxynicotinamide (LDD-3380)

¹H NMR (CDCl₃, 400 MHz): δ 8.48 (ddd, *J* = 7.6, 2.1, 0.8 Hz, 1 H), 8.28 (dq, *J* = 4.8, 0.8 Hz, 1 H), 7.29 (d, *J* = 8.5 Hz, 2 H), 7.22 (d, *J* = 8.9 Hz, 2 H), 6.44 (s, 1 H), 5.50 (s, 1 H), 5.20 (t, *J* = 5.6 Hz, 1 H), 4.05 (s, 3 H), 3.63 - 3.71 (m, 2 H), 3.52 - 3.61 (m, 2 H), 2.49 ppm (s, 3 H);
[M + H]⁺ = 445.15.

### Compound 8d: N-(2-(6-(4-bromophenylamino)-2-(methylthio)pyrimidin-4-ylamino)ethyl)-2-methoxynicotinamide (LDD-3381)

¹H NMR (CDCl₃, 400 MHz): δ 8.46 - 8.52 (m, 1 H), 8.26 - 8.31 (m, 1 H), 8.16 (t, *J* = 5.2 Hz, 1 H), 7.40 - 7.47 (m, 2 H), 7.14 - 7.21 (m, 2 H), 6.45 (s, 1 H), 5.51 (s, 1 H), 5.22 (t, *J* = 5.5 Hz, 1 H), 4.05 (s, 3 H), 3.67 (q, *J* = 5.9 Hz, 2 H), 3.52 - 3.62 (m, 2 H), 2.49 ppm (s, 3 H);
[M + H]⁺ = 491.90.

### Compound 8e: N-(2-(6-(4-iodophenylamino)-2-(methylthio)pyrimidin-4-ylamino)ethyl)-2-methoxynicotinamide (LDD-3382)

¹H NMR (CDCl₃, 400 MHz): δ 8.45 - 8.51 (m, 1 H), 8.27 (dt, *J* = 4.9, 1.2 Hz, 1 H), 8.16 (t, *J* = 5.6 Hz, 1 H), 7.57 - 7.65 (m, 2 H), 7.02 - 7.11 (m, 3 H), 6.45 (s, 1 H), 5.53 (s, 1 H), 5.22 (t, *J* = 5.5 Hz, 1 H), 4.05 (s, 3 H), 3.67 (q, *J* = 5.9 Hz, 2 H), 3.52 - 3.62 (m, 2 H), 2.49 ppm (s, 3 H);
[M + H]⁺ = 537.17.

### Compound 8f: N-(2-(6-(3,4-difluorophenylamino)-2-(methylthio)pyrimidin-4-ylamino)ethyl)-2-methoxynicotinamide (LDD-3399)

¹H NMR (CDCl₃, 400 MHz): δ 8.48 (dd, *J* = 7.5, 2.0 Hz, 1 H), 8.28 (dd, *J* = 4.9, 2.1 Hz, 1 H), 8.14 - 8.21 (m, 1 H), 7.28 - 7.33 (m, 1 H), 7.05 - 7.14 (m, 2 H), 6.90 - 6.96 (m, 1 H), 6.43 (s, 1 H), 5.47 (s, 1 H), 5.24 (t, *J* = 5.5 Hz, 1 H), 4.06 (s, 3 H), 3.67 (q, *J* = 6.2 Hz, 2 H), 3.53 - 3.61 (m, 2 H), 2.50 ppm (s, 3 H);
[M + H]⁺ = 447.24.

### Compound 8g: N-(2-(6-(2-chloro-4-fluorophenylamino)-2-(methylthio)pyrimidin-4-ylamino)ethyl)-2-methoxynicotinamide (LDD-3401)

¹H NMR (CDCl₃, 400 MHz): δ 8.48 (dd, *J* = 7.5, 2.0 Hz, 1 H), 8.28 (dd, *J=* 4.1, 2.0 Hz, 1 H), 8.17 (t, *J* = 5.3 Hz, 1 H), 7.78 (dd, J=9.2, 5.5 Hz, 1 H), 7.17 (dd, *J* = 8.2, 2.7 Hz, 1 H), 7.06 (dd, *J* = 7.3, 4.9 Hz, 1 H), 6.99 (td, *J* = 8.5, 2.7 Hz, 1 H), 6.52 (s, 1 H), 5.40 (s, 1 H), 5.24 - 5.32 (m, 1 H), 4.06 (s, 3 H), 3.68 (q, *J* = 5.7 Hz, 2 H), 3.54 - 3.64 (m, 2 H), 2.49 ppm (s, 3 H);
[M + H]⁺ = 463.22.

### Compound 9a: 2-methoxy-N-(2-(2-(methylsulfmyl)-6-(phenylamino)pyrimidin-4-ylamino)ethyl)nicotinamide (LDD-4098)

¹H NMR (CDCl₃, 400 MHz): δ 8.47 (dd, *J* = 2.06, 7.56 Hz, 1H), 8.29 (dd, *J* = 2.06, 5.04 Hz, 1H), 8.19 (t, *J* = 5.95 Hz, 1H), 7.36 - 7.41 (m, 2H), 7.24 (d, *J* = 8.01 Hz, 3H), 7.16 - 7.21 (m, 1H), 7.07 (dd, *J* = 4.81, 7.56 Hz, 1H), 6.87 (s, 1H), 5.77 (s, 1H), 5.66 (t, *J* = 4.81 Hz, 1H), 4.08 (s, 3H), 3.65 - 3.71 (m, 3H), 3.56 (br. s., 2H), 2.89 (s, 3H);
[M + H]⁺ = 426.98.

### Compound 9b: N-(2-(6-(4-fluorophenylamino)-2-(methylsulfinyl)pyrimidin-4-ylamino)ethyl)-2-methoxynicotinamide (LDD-4099)

¹H NMR (CDCl₃, 400 MHz): δ 8.47 (dd, *J* = 2.06, 7.56 Hz, 1H), 8.29 (dd, *J* = 2.06, 4.81 Hz, 1H), 8.19 (t, *J* = 5.04 Hz, 1H), 7.19 - 7.24 (m, 2H), 7.05 - 7.10 (m, 3H), 6.80 (s, 1H), 5.69 (t, *J* = 5.50 Hz, 1H), 5.62 (s, 1H), 4.08 (s, 3H), 3.67 (q, *J=* 6.03 Hz, 2H), 3.54 (br. s., 2H), 2.88 (s, 3H);
[M + H]⁺ = 444.98.

### Compound 9c: N-(2-(6-(4-chlorophenylamino)-2-(methylsulfinyl)pyrimidin-4-ylamino)ethyl)-2-methoxynicotinamide (LDD-4100)

¹H NMR (CDCl₃, 400 MHz): δ 8.45 (dd, *J* = 2.06, 7.56 Hz, 1H), 8.27 (dd, *J* = 2.06, 4.81 Hz, 1H), 8.18 (t, *J* = 5.72 Hz, 1H), 7.29 - 7.33 (m, 2H), 7.16 - 7.21 (m, *J* = 8.70 Hz, 2H), 7.05 (dd, *J* = 4.81, 7.56 Hz, 1H), 6.88 (br. s., 1H), 5.72 - 5.77 (m, 2H), 4.06 (s, 3H), 3.63 - 3.69 (m, 2H), 3.54 (br. s., 2H), 2.86 (s, 3H);
[M + H]⁺ = 460.94.

### Compound 9d: N-(2-(6-(4-bromophenylamino)-2-(methylsulfinyl)pyrimidin-4-ylamino)ethyl)-2-methoxynicotinamide (LDD-4101)

¹H NMR (CDCl₃, 400 MHz): δ 8.47 (dd, *J* = 1.83, 7.56 Hz, 1H), 8.29 (dd, *J* = 1.95, 4.92 Hz, 1H), 8.21 (t, *J* = 6.07 Hz, 1H), 7.46 - 7.50 (m, 3H), 7.16 (d, *J* = 8.70 Hz, 3H), 7.08 (dd, *J* = 4.81, 7.56 Hz, 1H), 6.89 (br. s., 1H), 5.77 (s, 2H), 4.08 (s, 4H), 3.65 - 3.71 (m, 3H), 3.56 (br. s., 2H), 2.88 (s, 3H);
[M + H]⁺ = 506.79.

### Compound 9e: N-(2-(6-(4-iodophenylamino)-2-(methylsulfinyl)pyrimidin-4-ylamino)ethyl)-2-methoxynicotinamide (LDD-3414)

¹H NMR (CDCl₃, 400 MHz): δ 8.48 (dd, *J* = 2.14, 7.63 Hz, 1H), 8.29 (dd, *J* = 2.14, 4.88 Hz, 1H), 8.20 (t, *J* = 5.49 Hz, 1H), 7.64 - 7.70 (m, 2H), 7.08 (dd, *J* = 4.73, 7.48 Hz, 1H), 7.02 - 7.06 (m, *J* = 8.55 Hz, 2H), 6.82 (s, 1H), 5.73 - 5.81 (m, 2H), 4.08 (s, 3H), 3.69 (q, *J* = 6.10 Hz, 2H), 3.57 (br. s., 2H), 2.88 (s, 3H);
[M + H]⁺ = 533.09.

### Compound 9f: N-(2-(6-(3,4-difluorophenylamino)-2-(methylsulfinyl)pyrimidin-4-ylamino)ethyl)-2-methoxynicotinamide (LDD-3416)

¹H NMR (CDCl₃, 400 MHz): δ 8.47 (dd, *J* = 7.5, 2.0 Hz, 1 H), 8.29 (dd, *J* = 4.7, 2.0 Hz, 1 H), 8.22 (t, *J* = 6.0 Hz, 1 H), 7.11 - 7.19 (m, 1 H), 7.08 (dd, *J* = 7.6, 4.9 Hz, 1 H), 6.94 - 7.01 (m, 1 H), 5.84 (t, *J* = 5.2 Hz, 1 H), 5.74 (s, 1 H), 4.09 (s, 2 H), 3.64 - 3.71 (m, 2 H), 3.56 (br. s., 1 H), 2.88 ppm (s, 2 H);
[M + H]⁺ = 463.22.

### Compound 9g: N-(2-(6-(3,4-difluorophenylamino)-2-(methylsulfonyl)pyrimidin-4-ylamino)ethyl)-2-methoxynicotinamide (LDD-3417)

¹H NMR (CDCl₃, 400 MHz): δ 8.47 (dd, *J* = 7.5, 2.0 Hz, 1 H), 8.30 (dd, *J* = 4.7, 2.0 Hz, 1 H), 8.20 - 8.28 (m, 1 H), 7.14 - 7.22 (m, 1 H), 7.09 (dd, *J* = 7.8, 4.7 Hz, 1 H), 6.95 - 7.01 (m, 1 H), 5.79 (br. s., 1 H), 5.35 ppm (s, 1 H);
[M + H]⁺ = 479.16.

### Compound 9h: N-(2-(6-(2-chloro-4-fluorophenylamino)-2-(methylsulfmyl)pyrimidin-4-ylamino)ethyl)-2-methoxynicotinamide (LDD-3418)

¹H NMR (CDCl₃, 400 MHz): δ 8.47 (dd, *J* = 7.3, 1.8 Hz, 1 H), 8.29 (dd, *J* = 4.9, 2.1 Hz, 1 H), 8.17 - 8.26 (m, 1 H), 7.61 - 7.69 (m, 1 H), 7.21 (dd, *J* = 7.9, 2.7 Hz, 1 H), 7.02 - 7.10 (m, 2 H), 6.77 (s, 1 H), 5.78 (t, *J* = 4.3 Hz, 1 H), 5.61 (s, 1 H), 4.08 (s, 3 H), 3.66 - 3.72 (m, 2 H), 3.54 - 3.61 (m, 2 H), 2.89 ppm (s, 3 H);
[M + H]⁺ = 479.15.

### Compound 9i: N-(2-(6-(2-chloro-4-fluorophenylamino)-2-(methylsulfonyl)pyrimidin-4-ylamino)ethyl)-2-methoxynicotinamide (LDD-3419)

¹H NMR (CDCl₃, 400 MHz): δ 8.47 (dd, *J* = 7.5, 2.0 Hz, 1 H), 8.30 (dd, *J* = 4.9, 1.8 Hz, 1 H), 8.22 (br. s., 1 H), 7.62 (td, *J* = 7.6, 2.6 Hz, 1 H), 7.22 (dd, *J* = 7.9, 3.1 Hz, 1 H), 7.02 - 7.10 (m, 3 H), 6.73 (br. s., 1 H), 5.90 (br. s., 1 H), 5.68 (br. s., 1 H), 5.36 (s, 1 H), 4.10 (s, 3 H), 3.67 - 3.72 (m, 2 H), 3.60 (br. s., 2 H), 3.25 ppm (s, 3 H);
[M + H]⁺ = 495.17.

### Compound 10a: (6-chloro-N-(4-fluorobenzyl)-2-(methylthio)-N-(4-morpholinophenyl)pyrimidin-4-amine) (LDD-3303)

¹H NMR (CDCl₃, 400 MHz): δ 7.17 - 7.24 (m, 2 H), 6.88 - 7.00 (m, 6 H), 5.80 (s, 1 H), 5.11 (s, 2 H), 3.85 - 3.92 (m, 4 H), 3.17 - 3.24 (m, 4 H), 2.51 ppm (s, 3 H);
[M + H]⁺ = 444.99.

### Compound 10b: 6-chloro-N-(4-fluorophenethyl)-2-(methylthio)-N-(4-morpholinophenyl)pyrimidin-4-amine (LDD-3316)

¹H NMR (CDCl₃, 400 MHz): δ 7.10 - 7.17 (m, 2 H), 6.93 - 7.02 (m, 6 H), 5.77 (s, 1 H), 4.05 - 4.12 (m, 2 H), 3.86 - 3.94 (m, 4 H), 3.20 - 3.26 (m, 4 H), 2.91 - 2.98 (m, 2 H), 2.58 ppm (s, 3 H);
[M + H]⁺ = 458.91.

### Compound 11a: N⁴-(4-fluorobenzyl)-2-(methylthio)-N⁴-(4-morpholinophenyl)pyrimidine-4,6-diamine (LDD-3304)

¹H NMR (CDCl₃, 400 MHz): δ 7.22 (dd, *J* = 8.5, 5.5 Hz, 2 H), 6.91 - 6.98 (m, 4 H), 6.85 - 6.89 (m, 2 H), 5.11 (s, 2 H), 4.91 (s, 1 H), 4.32 (s, 2 H), 3.83 - 3.90 (m, 4 H), 3.14 - 3.20 (m, 4 H), 2.47 ppm (s, 3 H);
[M + H]⁺ = 426.01.

### Compound 11b: N⁴-(4-fluorophenethyl)-2-(methylthio)-N⁴-(4-morpholinophenyl)pyrimidine-4,6-diamine (LDD-3319)

¹H NMR (CDCl₃, 400 MHz): δ 7.11 - 7.16 (m, 2 H), 6.91 - 7.05 (m, 6 H), 4.87 (s, 1 H), 4.33 (s, 2 H), 4.03 - 4.11 (m, 2 H), 3.85 - 3.92 (m, 4 H), 3.18 - 3.24 (m, 3 H), 2.90 - 2.98 (m, 2 H), 2.57 ppm (s, 3 H);
[M + H]⁺ = 439.98.

### Compound 13: (2-methoxypyridin-3-yl)(4-(2-(methylthio)-6-(4-morpholinophenylamino)pyrimidin-4-yl)piperazin-1-yl)methanone (LDD-3368)

¹H NMR (CDCl₃, 400 MHz): δ 8.23 (dd, *J* = 5.0, 2.0 Hz, 1 H), 7.61 (dd, *J* = 7.2, 2.0 Hz, 1 H), 7.13 - 7.21 (m, *J* = 8.2 Hz, 2 H), 6.96 (dd, *J* = 7.2, 5.0 Hz, 1 H), 6.88 - 6.94 (m, *J* = 8.5 Hz, 2 H), 6.44 (br. s., 1 H), 5.44 (s, 1 H), 3.97 (s, 3 H), 3.76 - 3.91 (m, 6 H), 3.55 - 3.64 (m, 4 H), 3.23 - 3.40 (m, 2 H), 3.16 (br. s., 4 H), 2.49 ppm (s, 3 H);
[M + H]⁺ = 522.13.

### Compound 14: (2-methoxypyridin-3-yl)(4-(2-(methylsulfinyl)-6-(4-morpholinophenylamino)pyrimidin-4-yl)piperazin-1-yl)methanone (LDD-3369)

¹H NMR (CDCl₃, 400 MHz): δ 8.21 - 8.26 (m, 1 H), 7.62 (dd, *J* = 6.9, 2.6 Hz, 1 H), 7.13 - 7.21 (m, 2 H), 6.91 - 7.00 (m, 3 H), 6.79 (s, 1 H), 5.62 (s, 1 H), 3.97 (s, 3 H), 3.80 - 3.93 (m, 6 H), 3.61 (br. s., 5 H), 3.32 (br. s., 2 H), 3.15 - 3.23 (m, 4 H), 2.88 ppm (s, 3 H);
[M + H]⁺ = 538.20.

### Compound 16a: 6-chloro-N-(2,4-difluorophenyl)-2-(methylsulfonyl)pyrimidin-4-amine (LDD-3379)

¹H NMR (CDCl₃, 400 MHz): δ 7.64 (br. s., 1 H), 7.13 (br. s., 1 H), 6.98 - 7.04 (m, 2 H), 6.62 (s, 1 H), 3.33 ppm (s, 3 H);
[M + H]⁺ = 320.22.

### Compound 16b: 6-chloro-2-(methylsulfonyl)-N-(3,4,5-trifluorophenyl)pyrimidin-4-amine (LDD-3582)

¹H NMR (CDCl₃, 400 MHz): δ 7.11 (dd, *J* = 7.7, 6.5 Hz, 2 H), 6.77 (s, 1 H), 3.34 ppm (s, 3 H);
[M + H]⁺ = 426.01;
[M - H]⁻ = 335.81.

### Compound 18a: N⁴,N⁶-bis(4-fluorophenyl)-2-(methylsulfonyl)pyrimidine-4,6-diamine (LDD-3365)

¹H NMR (CDCl₃, 400 MHz): δ 7.17 - 7.23 (m, 4 H), 7.04 - 7.11 (m, 4 H), 6.80 (s, 2 H), 5.85 (s, 1 H), 3.27 ppm (s, 3 H);
[M + H]⁺ = 376.88.

### Compound 18b: 2-(methylsulfonyl)-N⁴,N⁶-bis(3,4,5-trifluorophenyl)pyrimidine-4,6-diamine (LDD-3580)

¹H NMR (CDCl₃, 400 MHz): δ 7.03 (dd, *J* = 8.7, 6.0 Hz, 4 H), 6.88 (s, 2 H), 6.02 (s, 1 H), 3.29 ppm (s, 3 H);
[M + H]⁺ = 448.83.

### Compound 18c: N⁴,N⁶-bis(3-chloro-4-fluorophenyl)-2-(methylsulfonyl)pyrimidine-4,6-diamine (LDD-3581)

¹H NMR (CDCl₃, 400 MHz): δ 7.39 (dd, *J* = 6.9, 2.1 Hz, 2 H), 7.14 - 7.20 (m, 4 H), 6.81 (s, 2 H), 5.91 (s, 1 H), 3.28 ppm (s, 3 H);
[M + H]⁺ = 444.75.

### Compound 22a: tert-butyl 4-(6-(4-fluorophenylamino)-2-(methylsulfonyl)pyrimidin-4-yl)piperazine-1-carboxylate (LDD-3403)

¹H NMR (CDCl₃, 400 MHz): δ 7.22 - 7.26 (m, 2 H), 7.09 - 7.15 (m, 2 H), 6.79 (s, 1 H), 5.68 (s, 1 H), 3.48 - 3.60 (m, 8 H), 3.26 (s, 3 H), 1.47 ppm (s, 9 H);
[M - H]⁻ = 450.27.

### Compound 22b: tert-butyl 4-(6-(4-fluorobenzylamino)-2-(methylsulfonyl)pyrimidin-4-yl)piperazine-1-carboxylate (LDD-3409)

¹H NMR (CDCl₃, 400 MHz): δ 7.28 - 7.31 (m, 2 H), 7.05 (t, *J* = 8.7 Hz, 2 H), 5.37 (s, 1 H), 4.46 (d, *J* = 5.8 Hz, 2 H), 3.57 (br. s., 4 H), 3.51 (d, *J* = 5.8 Hz, 4 H), 3.22 (s, 3 H), 1.48 ppm (s, 9 H);
[M - H]⁻ = 464.13.

### Compound 22c: tert-butyl 4-(6-(4-fluorobenzylamino)-2-(methylsulfonyl)pyrimidin-4-yl)piperazine-1-carboxylate (LDD-3447)

¹H NMR (CDCl₃, 400 MHz): δ 7.36 - 7.39 (m, 2 H), 7.21 - 7.24 (m, 2 H), 6.80 (s, 1 H), 5.79 (s, 1 H), 3.59 (br. s., 4 H), 3.50 - 3.54 (m, 4 H), 3.26 (s, 3 H), 1.48 ppm (s, 9 H);
[M - H]⁻ = 466.10.

### Compound 22d: tert-butyl 4-(6-(4-chlorobenzylamino)-2-(methylsulfonyl)pyrimidin-4-yl)piperazine-1-carboxylate (LDD-3477)

¹H NMR (CDCl₃, 400 MHz): δ 7.32 - 7.35 (m, 2 H), 7.24 - 7.27 (m, 2 H), 5.44 (br. s., 1 H), 5.36 (s, 1 H), 4.47 (d, *J* = 5.5 Hz, 2 H), 3.57 (br. s., 4 H), 3.48 - 3.53 (m, 4 H), 3.21 (s, 3 H), 1.48 ppm (s, 9 H);
[M - H]⁻ = 480.00.

### Compound 22e: tert-butyl 4-(6-(4-bromophenylamino)-2-(methylsulfonyl)pyrimidin-4-yl)piperazine-1-carboxylate (LDD-3448)

¹H NMR (CDCl₃, 400 MHz): δ 7.48 - 7.52 (m, 2 H), 7.16 - 7.20 (m, 2 H), 6.91 (s, 1 H), 5.81 (s, 1 H), 3.58 (br. s., 4 H), 3.49 - 3.54 (m, 4 H), 3.25 (s, 3 H), 1.48 ppm (s, 9 H);
[M - H]⁻ = 511.95.

### Compound 22f: tert-butyl 4-(6-(4-iodophenylamino)-2-(methylsulfonyl)pyrimidin-4-yl)piperazine-1-carboxylate (LDD-3449)

¹H NMR (CDCl₃, 400 MHz): δ 7.68 - 7.71 (m, 2 H), 7.04 - 7.08 (m, 2 H), 6.86 (s, 1 H), 5.83 (s, 1 H), 3.59 (br. s., 4 H), 3.50 - 3.54 (m, 4 H), 3.25 (s, 3 H), 1.48 ppm (s, 9 H);
[M - H]⁻ = 557.97.

### Compound 22g: tert-butyl 4-(6-(3,4-difluorophenylamino)-2-(methylsulfonyl)pyrimidin-4-yl)piperazine-1-carboxylate (LDD-3451)

¹H NMR (CDCl₃, 400 MHz): δ 7.16 - 7.22 (m, 2 H), 6.98 - 7.03 (m, 1 H), 6.75 (s, 1 H), 5.74 (s, 1 H), 3.60 (br. s., 4 H), 3.51 - 3.55 (m, 4 H), 3.26 (s, 3 H), 1.48 ppm (s, 9 H);
[M - H]⁻ = 468.11.

### Compound 22h: tert-butyl 4-(2-(methylsulfonyl)-6-(3,4,5-trifluorophenylamino)pyrimidin-4-yl)piperazine-1-carboxylate (LDD-3505)

¹H NMR (CDCl₃, 400 MHz): δ 6.99 - 7.05 (m, 2 H), 6.77 (s, 1 H), 5.80 (s, 1 H), 3.63 (br. s., 4 H), 3.52 - 3.57 (m, 4 H), 3.27 (s, 3 H), 1.49 ppm (s, 9 H);
[M - H]⁻ = 485.99.

### Compound 22i: tert-butyl 4-(2-(methylsulfonyl)-6-(3,4,5-trifluorobenzylamino)pyrimidin-4-yl)piperazine-1-carboxylate (LDD-3450)

¹H NMR (CDCl₃, 400 MHz): δ 6.93 - 6.98 (m, 2 H), 5.40 (br. s., 1 H), 5.36 (s, 1 H), 4.48 (d, *J* = 5.7 Hz, 2 H), 3.57 (br. s., 4 H), 3.49 - 3.54 (m, 4 H), 3.21 (s, 3 H), 1.48 ppm (s, 9 H);
[M - H]⁻ = 500.10.

### Compound 26a: N-(4-fluorophenyl)-2-(methylsulfonyl)-6-(piperazin-1-yl)pyrimidin-4-amine (LDD-3404)

¹H NMR (CDCl₃, 400MHz): δ 7.20 - 7.24 (m, 2 H), 7.07 - 7.13 (m, 2 H), 6.73 - 6.79 (m, 1 H), 5.67 (s, 1 H), 3.54 (br. s., 4 H), 3.24 (s, 3 H), 2.90 - 2.94 ppm (m, 4 H);
[M + H]⁺ = 352.03.

### Compound 26b: 2-(methylsulfonyl)-6-(piperazin-1-yl)-N-(3,4,5-trifluorophenyl)pyrimidin-4-amine (LDD-3629)

¹H NMR (DMSO-*d*₆, 400MHz): δ 10.10 (s, 1 H), 7.54 (d, *J* = 6.2 Hz, 1 H), 7.56 (d, *J* = 6.4 Hz, 1 H), 6.13 (s, 1 H), 3.70 (br. s., 4 H), 3.31 (s, 3 H), 3.09 ppm (br. s., 4 H);
[M + H]⁺ = 387.87.

### Compound 26c: N-(3-chloro-4-fluorophenyl)-2-(methylsulfonyl)-6-(piperazin-1-yl)pyrimidin-4-amine (LDD-3666)

¹H NMR (DMSO-*d*₆, 400MHz): δ 9.80 (s, 1 H), 7.89 (dd, *J* = 6.8, 2.6 Hz, 1 H), 7.46 (ddd, *J* = 9.0, 4.3, 2.7 Hz, 1 H), 7.36 (t, *J* = 9.0 Hz, 1 H), 5.99 (s, 1 H), 3.44 - 3.51 (m, 4 H), 3.27 (s, 3 H), 2.73 - 2.78 ppm (m, 4 H);
[M - H]⁻ = 383.88.

### Compound 26d: N-(3-bromo-4-fluorophenyl)-2-(methylsulfonyl)-6-(piperazin-1-yl)pyrimidin-4-amine (LDD-3682)

¹H NMR (DMSO-*d*₆, 400MHz): δ 9.70 (s, 1 H), 8.00 (dd, *J* = 6.4, 2.8 Hz, 1 H), 7.49 (ddd, *J* = 8.9, 4.4, 2.8 Hz, 1 H), 7.33 (t, *J* = 8.7 Hz, 1 H), 5.95 (s, 1 H), 3.45 - 3.49 (m, 4 H), 3.27 (s, 3 H), 2.74 - 2.77 ppm (m, 4 H);
[M + H]⁺ = 431.86.

### Compound 26e: 2-(methylsulfonyl)-N⁴-(piperidin-4-yl)-N⁶-(3,4,5-trifluorophenyl)pyrimidine-4,6-diamine (LDD-3644)

¹H NMR (DMSO-*d*₆, 400MHz): δ 9.87 (br. s., 1 H), 7.84 (br. s., 1 H), 7.46 - 7.54 (m, 2 H), 5.93 (br. s., 1 H), 4.08 (br. s., 2 H), 3.29 (s, 3 H), 2.97 - 3.06 (m, 2 H), 2.04 (d, *J* = 12.8 Hz, 2 H), 1.55 - 1.67 ppm (m, 2 H);
[M + H]⁺ = 401.90.

### Compound 26f: N⁴-(3-chloro-4-fluorophenyl)-2-(methylsulfonyl)-N⁶-(piperidin-4-yl)pyrimidine-4,6-diamine (LDD-3678)

¹H NMR (DMSO-*d*₆, 400MHz): δ 9.58 (br. s., 1 H), 7.83 (br. s., 1 H), 7.59 (br. s., 1 H), 7.34 - 7.42 (m, 2 H), 5.84 (br. s., 1 H), 3.31 (br. s., 3 H), 2.94 (d, *J* = 12.6 Hz, 2 H), 1.81 (d, *J* = 9.6 Hz, 2 H), 1.25 - 1.35 ppm (m, 2 H);
[M + H]⁺ = 399.81.

### Compound 26g: N⁴-(3-bromo-4-fluorophenyl)-2-(methylsulfonyl)-N⁶-(piperidin-4-yl)pyrimidine-4,6-diamine (LDD-3683)

¹H NMR (DMSO-*d*₆, 400MHz): δ 9.56 (br. s., 1 H), 7.93 (br. s., 1 H), 7.54 - 7.63 (m, 1 H), 7.42 - 7.48 (m, 1 H), 7.33 (t, *J* = 8.8 Hz, 1 H), 5.83 (br. s., 1 H), 3.81 (br. s., 1 H), 3.24 (s, 3 H), 2.94 (d, *J* = 12.4 Hz, 2 H), 1.81 (d, *J* = 11.2 Hz, 2 H), 1.29 ppm (d, *J* = 8.9 Hz, 2 H);
[M + H]⁺ = 445.86.

### Compound 26h: 6-(4-aminopiperidin-1-yl)-2-(methylsulfonyl)-N-(3,4,5-trifluorophenyl)pyrimidin-4-amine (LDD-3705)

¹H NMR (DMSO-*d*₆, 400MHz): δ 9.98 (s, 1 H), 7.51 - 7.56 (m, 2 H), 6.05 (s, 1 H), 3.61 (br. s., 4 H), 3.29 (s, 3 H), 2.94 ppm (br. s., 4 H);
[M - H]⁻ = 399.96.

### Compound 26i: 6-(4-aminopiperidin-1-yl)-N-(3-chloro-4-fluorophenyl)-2-(methylsulfonyl)pyrimidin-4-amine (LDD-3677)

¹H NMR (DMSO-*d*₆, 400MHz): δ 9.70 (br. s., 1 H), 7.89 (dd, *J* = 6.8, 2.6 Hz, 1 H), 7.42 - 7.47 (m, 1 H), 7.36 (t, *J* = 9.0 Hz, 1 H), 6.00 (s, 1 H), 4.10 (br. s., 2 H), 3.27 (s, 3 H), 3.04 (ddd, *J* = 13.5, 11.2, 2.7 Hz, 2 H), 2.80 - 2.89 (m, 1 H), 1.78 (dd, *J* = 12.6, 3.0 Hz, 2 H), 1.13 - 1.24 ppm (m, 2 H);
[M + H]⁺ = 399.80.

### Compound 26j: 6-(4-aminopiperidin-1-yl)-N-(3-bromo-4-fluorophenyl)-2-(methylsulfonyl)pyrimidin-4-amine (LDD-3684)

¹H NMR (DMSO-*d*₆, 400MHz): δ 9.67 (br. s., 1 H), 8.00 (dd, *J* = 6.3, 2.6 Hz, 1 H), 7.48 (ddd, *J* = 9.0, 4.2, 2.6 Hz, 1 H), 7.30 - 7.36 (m, 1 H), 5.99 (s, 1 H), 4.10 (br. s., 2 H), 3.27 (s, 3 H), 2.99 - 3.09 (m, 2 H), 2.82 - 2.91 (m, 1 H), 1.78 (d, *J* = 15.4 Hz, 2 H), 1.14 - 1.26 ppm (m, 2 H);
[M - H]⁻ = 443.82.

### Compound 4925: 6-((1R,4R)-2,5-diazabicyclo[2.2.1]heptan-2-yl)-N-(3-chloro-4-fluorophenyl)-2-(methylsulfonyl)pyrimidin-4-amine (LDD-4925)

¹H NMR (DMSO-*d*₆, 400MHz): δ 1.64 - 1.83 (m, 2 H) 2.72 - 2.79 (m, 1 H) 2.90 (d, *J* = 8.24 Hz, 1 H) 3.03 - 3.12 (m, 1 H) 3.27 (s, 3 H) 3.45 (m, 1 H) 3.63 - 3.74 (m, 1 H) 4.30 - 4.40 (br. s., 0.25 H) 4.85 - 4.94 (br. s., 0.5 H) 5.54 - 5.65 (br. s., 0.5 H) 5.80 - 5.90 (br. s., 0.25 H) 7.36 (t, *J* = 9.04 Hz, 1 H) 7.44 (ddd, *J* = 9.04, 4.24, 2.52 Hz, 1 H) 7.89 (dd, *J* = 6.75, 2.63 Hz, 1 H) 9.66 - 9.72 (m, 1 H);

The ¹H NMR spectra of the compound at room temperature displayed doubling of some signals, suggesting that two rotational conformers are existed in a ratio ∼ 1:1 in DMSO-d₆.
[M + H]⁺ = 398.5.

### Compound 4941: N-(3-chloro-4-fluorophenyl)-2-(methylsulfonyl)-6-(2,6-diazaspiro[3.3]heptan-2-yl)pyrimidin-4-amine (LDD-4941)

¹H NMR (METHANOL-*d*₄, 400MHz): δ 3.25 (s, 3 H) 4.31 (s, 4 H) 4.35 (s, 4 H) 5.66 (s, 1 H) 7.18 (t, *J* = 9.04 Hz, 1 H) 7.46 (ddd, *J* = 9.16, 4.12, 2.75 Hz, 1 H) 7.80 (dd, *J* = 6.64, 2.52 Hz, 1H);
[M + H]⁺ = 398.5.

### Compound 4942: 6-((1S,4S)-2,5-diazabicyclo[2.2.1]heptan-2-yl)-N-(3-chloro-4-fluorophenyl)-2-(methylsulfonyl)pyrimidin-4-amine (LDD-4942)

¹H NMR (CHLOROFORM-d, 400MHz): δ 1.84 (br. s., 2 H) 2.90 - 3.05 (m, 1 H) 3.05 - 3.16 (m, 2 H) 3.26 (s, 3 H) 3.39 (br. s., 1 H) 3.88 (br. s., 1 H) 5.11 (br. s., 1 H) 5.41 (br. s., 1 H) 6.79 (s, 1 H) 7.14 - 7.19 (m, 2 H) 7.34 - 7.40 (m, 1 H);
[M + H]⁺ = 398.4.

### Compound 4943: (1R,5S,6s)-3-(6-(3-chloro-4-fluorophenylamino)-2-(methylsulfonyl)pyrimidin-4-yl)-3-azabicyclo[3.1.0]hexan-6-amine (LDD-4943)

¹H NMR (ACETONE-*d*₆, 400MHz): δ 1.88 (br. s., 2 H) 2.33 (q, *J* = 2.14 Hz, 1 H) 3.23 (s, 3 H) 3.44 - 3.68 (m, 2 H) 3.83 (br. s., 2 H) 5.74 (s, 1 H) 7.26 (t, *J* = 9.04 Hz, 1 H) 7.55 (ddd, *J* = 9.16, 4.12, 2.75 Hz, 1 H) 7.92 (dd, *J* = 6.64, 2.75 Hz, 1 H);
[M + H]⁺ = 398.4.

### Experimental Example 1: Expression and purification of HBV core protein

Cp 149 (amino acids 1-149) is a truncated form of a HBV core protein. It was expressed in Escherichia coli, and the expressed protein was purified. The recombinant Cp149 was expressed in E. coli BL21(DE3) cells cultured in LB medium at 37°C. When the culture reached an OD600 of 0.7 to 0.8, expression of the culture was induced with 0.5 mM isopropyl-β-d-thiogalactopyranoside and the culture was cooled to 16°C. After 16 hours, the cells were harvested, flash-frozen in liquid nitrogen, and stored at -80°C. To purify Cp149, thawed cells were resuspended in lysis buffer (20 mM Tris-HCl pH 9.0, 200 mM NaCl, 10 mM imidazole, 10 µg per mL DNasel, 1 mM phenylmethylsulfonyl fluoride), and treated with ultrasonic waves. The supernatant was loaded onto Ni-NTA affinity resin. Proteins were eluted using a 20 mM to 500 mM imidazole gradient dissolved in lysis buffer. After removing the histidine tag with thrombin, the protein was further purified by HiTrap Q anion exchange chromatography (17115401, GE Healthcare).

### Experimental Example 2: Immunoblot analysis of in vitro HBV capsid assembly

To determine the effect on *in vitro* Cp149 assembly, compounds of the present invention were added to the Cp149-containing suspension. The final concentration of Cpl49 was 1 mg per mL, and the compounds of the present invention were added at concentrations of 0.1 to 10 µM. The suspension was mixed with reaction buffer (150 mM HEPES, pH 7.5, 15 mM NaCl) added in a 2:1 ratio. *In vitro* assembly was performed at 37° C for 1 hour, and HBV capsid was detected by immunoblot analysis using an anti-HBV core antibody (1:2000, B0586, Dako).

### Experimental Example 3: Sucrose density gradient analysis of in vitro HBV capsid assembly

Cp149 constructs formed by assembly in the presence and absence of inhibitory compounds were subjected to sucrose density gradient analysis. Sample (150 µL) was laid on a sucrose density gradient consisting of 800 µL of 50% (wt per vol, hereinafter the same was applied) sucrose which was dissolved in 150mM HEPES at pH7.5, 800 µL of 40% sucrose, 800 µL of 30% sucrose, 800 µL of 20% sucrose, and 650 µL of 10% sucrose. After centrifugation at 250,000 × g and 20° C for 1.5 hours, ten 400 µL fractions were collected from top to bottom, and each fraction was analyzed by 15% SDS-PAGE. The gels were stained with Coomassie Brilliant Blue R-250 or subjected to immunoblot analysis using an anti-HBV core antibody. The density of individual bands was analyzed with ImageJ software.

### Experimental Example 4: Cell viability

HepG2.2.15 cells were seeded in 96-well plates at a density of 2×10⁴ cells per well. After treatment with the indicated compounds, the medium was removed and the cells were cultured in EZ-CYTOX solution (EZ-5000, DoGenBio) for 1 hour at 37°C. Absorbance was measured at 450 nm.

### Experimental Example 5: Analysis of intracellular HBV capsid assembly

Huh-7 cells were seeded in a 6-well plate at a density of 5×10⁵ cells per well and transfected with pCDNA3-Core (genotype C) plasmid DNA. After 12 hours, the cells were incubated with the indicated compounds for 36 hours. These cells were lysed on ice with lysis buffer (1% NP40) for 10 minutes, and cell debris and nuclei were removed by centrifugation at 15,000 × g. The supernatant was loaded on a sucrose gradient consisting of 1 mL of 40% (wt per vol, hereinafter the same was applied) sucrose dissolved in 1 × PBS and 1.5 mL of 20% sucrose, and HBV capsids were precipitated by centrifugation for 8 hours at 400,000 × g and 20 ° C. The pellet was resuspended in 50 µL of 1×PBS, sonicated (1 s per stroke×3 times) and the samples separated on a 1% agarose gel. The gel was transferred to a nitrocellulose membrane by capillary transfer in 10×SSC, and HBV core particles were detected by immunoblot analysis using an anti-HBV core antibody (1:2000, B0586, Dako).

### Experimental Example 6: Quantitative PCR for measuring HBV DNA in cells

After treating cells with the indicated compounds, HBV DNA secreted into the medium and intracellular HBV DNA were extracted, purified, and quantified by quantitative PCR. To isolate the secreted HBV DNA, the culture medium was recovered and centrifuged at 15,000 × g to remove debris. The medium was diluted 1:1 with phosphate-buffered saline (PBS), and 1 M NaOH solution was added to a final concentration of 0.1 M. The mixture was incubated at 37° C for 1 hour. The protein was denatured by adding 2M Tris-HCl solution (pH 7.5) to a final concentration of 0.2 M and incubating at 98°C for 5 minutes. The protein precipitation was removed by centrifugation at 15,000 × g, and the supernatant was subjected to PCR with HBV DNA primer and probe sets:
Forward: 5'-TCCTCTTCATCCTGCTGCTATG-3', Reverse: 5'-CGTGCTGGTAGTTGATGTTCCT-3', Probe: 5'-TATTGGTTCTTCTGGACTA-3'.

**[TABLE 1]**

| 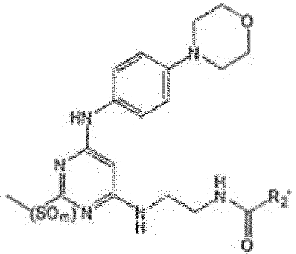 | | | | |
|---|---|---|---|---|
| **Compound (LDD Code)** | m | R₂' | Protein based assay (% inhibition 10 µM) | HBV DNA quantification (IC₅₀ µM) |
| 4a(LDD-3139) | 0 | 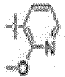 | 23 (IC₅₀=9A µM) | >10 |
| 4b(LDD-3345) | 0 | 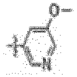 | 37 | >10 |
| 4c(LDD-31 57) | 0 | 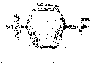 I | 0 | >10 |
| 4d(LDD-31SS) | 0 | 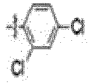 | 0 | >10 |
| 5a(LDD-3140) | 1 | 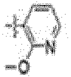 | 95 | 0.402 |
| | | | 43(0.1 µM) | |
| | | | 83(1 µM) | |
| | | | (IC₅₀=5.3 µM) | |
| 5b(LDD-3366) | 1 | 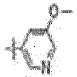 | 78 | >10 |
| 5c(LDD-3160) | 1 | 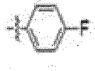 | 0 | >10 |
| 5d(LDD-3156) | 1 | 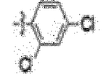 | 0 | >10 |

**[TABLE 2]**

| 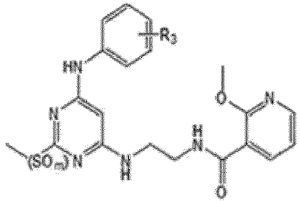 | | | | |
|---|---|---|---|---|
| **Compound (LDD Code)** | m | 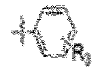 | Protein based assay (% inhibition, 10 µM) | HBV DNA quantification (IC₅₀µM) |
| 8a(LDD-3343) | 0 | 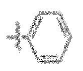 | 0 | >10 |
| 8b(LOD-3346) | 0 | 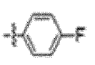 | 74 | >10 |
| 8c(LDD-3380) | 0 | 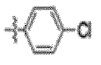 | 26 | >10 |
| 8d(LDD-3381) | 0 | 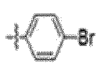 | 76 | >10 |
| 8e(LDO-3382) | 0 | 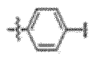 | 43 | >10 |
| 8f(LDD-3399) | 0 | 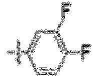 | 37 | >10 |
| 8g(LDD-3401) | 0 | 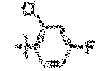 | 43 | >10 |

**[TABLE 3]**

| 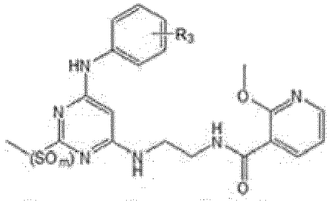 | | | | |
|---|---|---|---|---|
| **Compound (LDD Code)** | m | 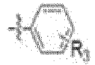 | Protein based assay (% inhibition, 10 µM) | HBV DNA quantification (IC₅₀ µM) |
| 9a(LDD-4098) | 1 | 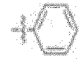 | 14(0.1 µM) | 0.484 |
| | | | 83(1 µM) | |
| 9b(LDD-4099) | 1 | 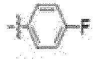 | 32(0.1 µM) | 0.210 |
| | | | 83(1 µM) | |
| 9c(LDD-4100) | 1 | 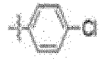 | 27(0.1 µM) | 0.299 |
| | | | 80(1 µM) | |
| 9d(LDD-4101) | 1 | 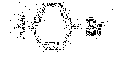 | 27(0.1 µM) | 0.177 |
| | | | 82(1 µM) | |
| 9e(LDD-3414) | 1 | 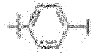 | 37 | >10 |
| 9f(LDD-3416) | 1 | 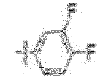 | 38 | >10 |
| 9g(LDD-3417) | 2 | 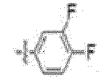 | 59 | >10 |
| 9h(LDD-3418) | 1 | 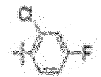 | 43 | >10 |
| 9i(LDD-3419) | 2 | 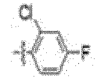 | 43 | >10 |

**[TABLE 4]**

| 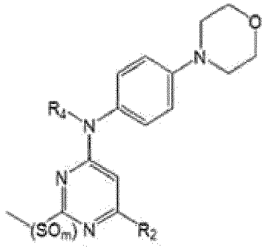 | | | | | |
|---|---|---|---|---|---|
| **Compound (LDD Code)** | m | R₂ | R₄ | Protein based assay (% inhibition, 10 pM) | HBV DNA quantification (IC₅₀ µM) |
| 10a(LDD-3303) | 0 | 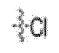 | 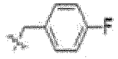 | 58 | >10 |
| 10b(LDD-3316) | 0 | 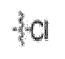 | 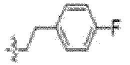 | 76 | >10 |
| 11a(LDD-3304) | 0 | 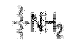 | 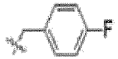 | 71 | >10 |
| 11b(LDD-3319) | 0 | 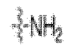 | 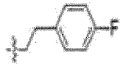 | 74 | >10 |
| 13(LDD-3368) | 0 | 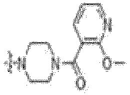 | 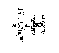 | 31 | >10 |
| 14(LDD-3369) | 1 | 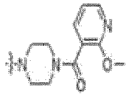 | 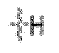 | 33 | >10 |

**[TABLE 5]**

| 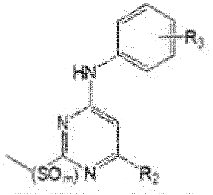 | | | | | |
|---|---|---|---|---|---|
| **Compound (LDD Code)** | m | R₂ | 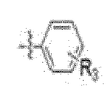 | Protein based assay (% inhibition, 10 µM) | HBV DNA quantification (IC₅₀ µM) |
| 16a(LDD-3179) | 2 | 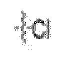 | 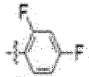 | 85 | >10 |
| 16b(LDD-3582) | 2 | 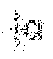 | 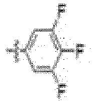 | 32 | 0.473 |
| 18a(LDD-3365) | 2 | 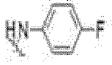 | 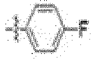 | 49 | 6.400 |
| 18b(LDD-3580) | 2 | 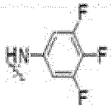 | 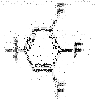 | 69 | 1.021 |
| 18c(LDD-3581) | 2 | 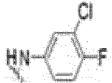 | 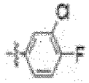 | 57 | 0.587 |

**[TABLE 6]**

| 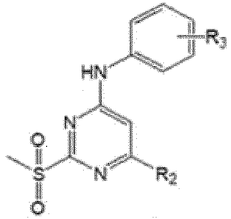 | | | | |
|---|---|---|---|---|
| **Compound (LDD Code)** | R₂ | 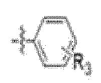 | Protein based assay (% inhibition, 10 µM) | HBV DNA quantification (IC₅₀ µM) |
| 22a(LDD-3403) | 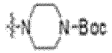 | 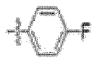 | 68 | 0.831 |
| 22b(LDD-3409) | 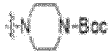 | 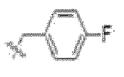 | 40 | 0.549 |
| 22c(LDD-3447) | 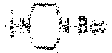 | 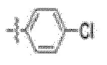 | 68 | 0.577 |
| 22d(LDD-3477) | 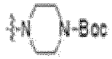 | 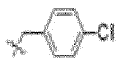 | 55 | 5.083 |
| 22e(LDD-3448) | 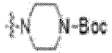 | 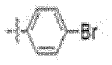 | 67 | 0.652 |
| 22f(LDD-3449) | 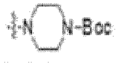 | 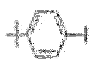 | 66 | 1.100 |
| 22g(LDD-3451) | 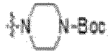 | 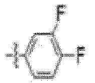 | 84 | 0.658 |
| 22h(LDD-3505) | 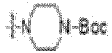 | 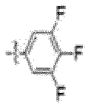 | 96 | 0.577 |
| 22i(LDD-3450) | 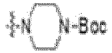 | 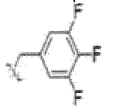 | 89 | 1.019 |

**[TABLE 7]**

| 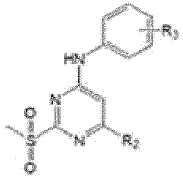 | | | | |
|---|---|---|---|---|
| **Compound (LDD Code)** | R₂ | 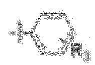 | Protein based assay (% inhibition. 10 µM) | H8V DNA quantification (IC₅₀ µM). |
| 26a(LDD-3404) | 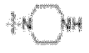 | 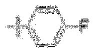 | 86 | 0.562 |
| 26b(LDD-3629) | 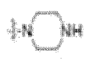 | 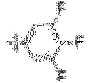 | 39(0.1 µM) | 0.203 |
| | | | 92(1 µM) | |
| 26c(LDD-3666) | 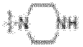 | 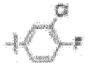 | 47(0.1 µM) | 0.189 |
| | | | 90(1 µM) | |
| 26d(LDD-3682) | 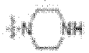 | 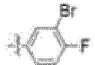 | 13(0.1 µM) | 0.547 |
| | | | 98(1 µM) | |
| 26e(LDD-3644) | 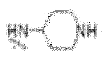 | 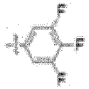 | 16(0.1 µM) | 0.190 |
| | | | 90(1 µM) | |
| 26f(LDD-3678) | 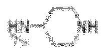 | 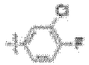 | 59(0.1 µM) | 0.199 |
| | | | 95(1 µM) | |
| 26g(LDD-3683) | 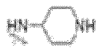 | 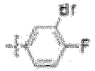 | 7(0.1 µM) | 0.536 |
| | | | 97(1 µM) | |
| 26h(LDE)-3705) | 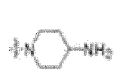 | 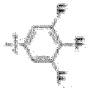 | 29(0.1 µM) | 0.292 |
| | | | 96(1 µM) | |
| 26i(LDD-3677) | 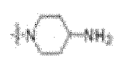 | 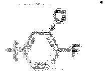 | 52(0.1 µM) | 0.181 |
| | | | | |
| 26j(LDD-3684) | 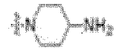 | 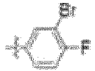 | 10(0.1 µM) | 0.480 |
| | | | 98(1 µM) | |
| 4925(LDD-4925) | 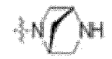 | 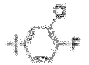 | - | 0.150 |
| 4941(LDD-4941) | 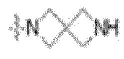 | 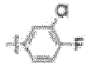 | - | 0.235 |
| 4942(LDD-4942) | 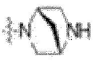 | 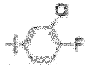 | - | 0.428 |
| 4943(LDD-4943) | 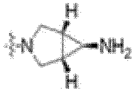 | 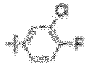 | - | 0.769 |

From the above description, those skilled in the art to which the present invention pertains will understand that the present invention can be embodied in other specific forms without changing the technical spirit or essential features thereof. In this regard, it should be understood that the embodiments described above are illustrative in all respects and not limiting. The scope of the present invention should be construed as including all changes or modifications derived from the meaning and scope of the claims to be described later and equivalent concepts rather than the detailed description above.

## Claims

1. A compound represented by Formula 1 or a pharmaceutically acceptable salt thereof : 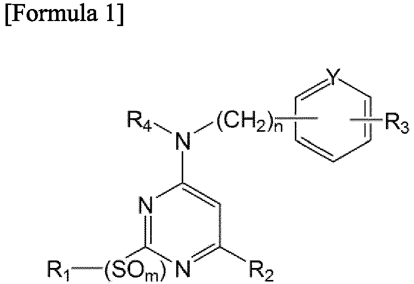 wherein,
m is 0, 1 or 2;
n is an integer from 0 to 4;
Yis C(H) or N;
R₁ is C₁₋₄ alkyl;
R₂ is hydrogen, halogen, amino, -NH-(CH₂)ₚ-NHCO-R₅, or
C₆₋₁₀ aryl, C₆₋₁₀ arylamino, 3-10 membered heterocyclyl, or 3-10 membered heterocyclylamino each unsubstituted or substituted by R₆;
R₅ is tert-butoxy, C₆₋₁₀ aryl or 5-10 membered heteroaryl;
R₆ is amino unsubstituted or substituted by tert-butoxycarbonyl, or tert-butoxy, C₆₋₁₀ aryl, or 5-10 membered heteroaryl linked directly or through - CO-,
p is an integer from 1 to 4;
R₃ is hydrogen, 3-10 membered heterocyclyl, cyano, C₁₋₄ alkyl, C₁₋₄ haloalkyl, or 1 to 3 halogens; and
R₄ is hydrogen, or C₆₋₁₀ aryl-C₁₋₄ alkyl;
wherein said aryl, heteroaryl and heterocyclyl are unsubstituted or substituted by one or more selected from halogen, C₁₋₄ alkyl, C₁₋₄ alkoxy, or amino.

2. The compound or a pharmaceutically acceptable salt thereof of claim 1,
wherein R₁ is methyl.

3. The compound or a pharmaceutically acceptable salt thereof of claim 1,
wherein R₂ is hydrogen, halogen, amino, -NH-(CH₂)₂-NHCO-R₅, or phenylamino, piperazinyl, piperidinyl, piperidinylamino, (1R,4R)-2,5-diazabicyclo[2.2.1]heptyl, (1S,4S)-2,5-diazabicyclo[2.2.1]heptyl, 2,6-diazaspiro[3.3]heptyl, or 3-azabicyclo[3.1.0]hexyl each unsubstituted or substituted by R₆;
R₅ is tert-butoxy, phenyl or pyridinyl; and
R₆ is amino unsubstituted or substituted by tert-butoxycarbonyl, or
tert-butoxy, phenyl or pyridinyl linked directly or through -CO-.

4. The compound or a pharmaceutically acceptable salt thereof of claim 1,
wherein R₂ is hydrogen, chloro, amino,
piperazinyl, piperidinylamino, or aminopiperidinyl each unsubstituted or substituted by butoxycarbonyl,
phenylamino, (methoxypyridinyl)carbonylpiperazinyl, or (methoxypyridinyl)carbonylaminoethylamino each substituted by 1 to 3 halogens selected from fluoro or chloro,
phenylcarbonylaminoethylamino substituted by 1 or 2 halogens selected from fluoro or chloro,
(1R,4R)-2,5-diazabicyclo[2.2.1]heptyl unsubstituted or substituted by butoxycarbonyl,
(1S,4S)-2,5-diazabicyclo[2.2.1]heptyl unsubstituted or substituted by butoxycarbonyl,
2,6-diazaspiro[3.3]heptyl unsubstituted or substituted by butoxycarbonyl, or
3-azabicyclo[3.1.0]hexyl unsubstituted or substituted by butoxycarbonyl or butoxycarbonylamino.

5. The compound or a pharmaceutically acceptable salt thereof of claim 1,
wherein R₃ is hydrogen, morpholinyl, cyano, methyl, trifluoromethyl, or 1 to 3 substituents selected from the group consisting of fluoro, chloro, bromo, and iodo.

6. The compound or a pharmaceutically acceptable salt thereof of claim 1,
wherein R₄ is hydrogen, benzyl or phenylethyl.

7. The compound or a pharmaceutically acceptable salt thereof of claim 1,
wherein the compound is,
(1) 2-methoxy-N-(2-(2-(methylthio)-6-(4-morpholinophenylamino)pyrimidin-4-ylamino)ethyl)nicotinamide,
(2) 5-methoxy-N-(2-(2-(methylthio)-6-(4-morpholinophenylamino)pyrimidin-4-ylamino)ethyl)nicotinamide,
(3) 4-fluoro-N-(2-(2-(methylthio)-6-(4-morpholinophenylamino)pyrimidin-4-ylamino)ethyl)benzamide,
(4) 2,4-dichloro-N-(2-(2-(methylthio)-6-(4-morpholinophenylamino)pyrimidin-4-ylamino)ethyl)benzamide,
(5) 2-methoxy-N-(2-(2-(methylsulfinyl)-6-(4-morpholinophenylamino)pyrimidin-4-ylamino)ethyl)nicotinamide,
(6) 5-methoxy-N-(2-(2-(methylsulfinyl)-6-(4-morpholinophenylamino)pyrimidin-4-ylamino)ethyl)nicotinamide,
(7) 4-fluoro-N-(2-(2-(methylsulfinyl)-6-(4-morpholinophenylamino)pyrimidin-4-ylamino)ethyl)benzamide,
(8) 2,4-dichloro-N-(2-(2-(methylsulfinyl)-6-(4-morpholinophenylamino)pyrimidin-4-ylamino)ethyl)benzamide,
(9) 2-methoxy-N-(2-(2-(methylthio)-6-(phenylamino)pyrimidin-4-ylamino)ethyl)nicotinamide,
(10) N-(2-(6-(4-fluorophenylamino)-2-(methylthio)pyrimidin-4-ylamino)ethyl)-2-methoxynicotinamide,
(11) N-(2-(6-(4-chlorophenylamino)-2-(methylthio)pyrimidin-4-ylamino)ethyl)-2-methoxynicotinamide,
(12) N-(2-(6-(4-bromophenylamino)-2-(methylthio)pyrimidin-4-ylamino)ethyl)-2-methoxynicotinamide,
(13) N-(2-(6-(4-iodophenylamino)-2-(methylthio)pyrimidin-4-ylamino)ethyl)-2-methoxynicotinamide
(14) N-(2-(6-(3,4-difluorophenylamino)-2-(methylthio)pyrimidin-4-ylamino)ethyl)-2-methoxynicotinamide,
(15) N-(2-(6-(2-chloro-4-fluorophenylamino)-2-(methylthio)pyrimidin-4-ylamino)ethyl)-2-methoxynicotinamide,
(16) 2-methoxy-N-(2-(2-(methylsulfinyl)-6-(phenylamino)pyrimidin-4-ylamino)ethyl)nicotinamide,
(17) N-(2-(6-(4-fluorophenylamino)-2-(methylsulfinyl)pyrimidin-4-ylamino)ethyl)-2-methoxynicotinamide,
(18) N-(2-(6-(4-chlorophenylamino)-2-(methylsulfinyl)pyrimidin-4-ylamino)ethyl)-2-methoxynicotinamide,
(19) N-(2-(6-(4-bromophenylamino)-2-(methylsulfinyl)pyrimidin-4-ylamino)ethyl)-2-methoxynicotinamide,
(20) N-(2-(6-(4-iodophenylamino)-2-(methylsulfinyl)pyrimidin-4-ylamino)ethyl)-2-methoxynicotinamide,
(21) N-(2-(6-(3,4-difluorophenylamino)-2-(methylsulfinyl)pyrimidin-4-ylamino)ethyl)-2-methoxynicotinamide,
(22) N-(2-(6-(3,4-difluorophenylamino)-2-(methylsulfonyl)pyrimidin-4-ylamino)ethyl)-2-methoxynicotinamide,
(23) N-(2-(6-(2-chloro-4-fluorophenylamino)-2-(methylsulfmyl)pyrimidin-4-ylamino)ethyl)-2-methoxynicotinamide,
(24) N-(2-(6-(2-chloro-4-fluorophenylamino)-2-(methylsulfonyl)pyrimidin-4-ylamino)ethyl)-2-methoxynicotinamide,
(25) 6-chloro-N-(4-fluorobenzyl)-2-(methylthio)-N-(4-morpholinophenyl)pyrimidin-4-amine,
(26) 6-chloro-N-(4-fluorophenethyl)-2-(methylthio)-N-(4-morpholinophenyl)pyrimidin-4-amine,
(27) N⁴-(4-fluorobenzyl)-2-(methylthio)-N⁴-(4-morpholinophenyl)pyrimidine-4,6-diamine,
(28) N⁴-(4-fluorophenethyl)-2-(methylthio)-N⁴-(4-morpholinophenyl)pyrimidine-4,6-diamine,
(29) (2-methoxypyridin-3-yl)(4-(2-(methylthio)-6-(4-morpholinophenylamino)pyrimidin-4-yl)piperazin-1-yl)methanone,
(30) (2-methoxypyridin-3-yl)(4-(2-(methylsulfinyl)-6-(4-morpholinophenylamino)pyrimidin-4-yl)piperazin-1-yl)methanone,
(31) 6-chloro-N-(2,4-difluorophenyl)-2-(methylsulfonyl)pyrimidin-4-amine,
(32) 6-chloro-2-(methylsulfonyl)-N-(3,4,5-trifluorophenyl)pyrimidin-4-amine,
(33) N⁴,N⁶-bis(4-fluorophenyl)-2-(methylsulfonyl)pyrimidine-4,6-diamine,
(34) 2-(methylsulfonyl)-N⁴,N⁶-bis(3,4,5-trifluorophenyl)pyrimidine-4,6-diamine,
(35) N⁴,N⁶-bis(3-chloro-4-fluorophenyl)-2-(methylsulfonyl)pyrimidine-4,6-diamine,
(36) tert-butyl 4-(6-(4-fluorophenylamino)-2-(methylsulfonyl)pyrimidin-4-yl)piperazine-1-carboxylate,
(37) tert-butyl 4-(6-(4-fluorobenzylamino)-2-(methylsulfonyl)pyrimidin-4-yl)piperazine-1-carboxylate,
(38) tert-butyl 4-(6-(4-chlorophenylamino)-2-(methylsulfonyl)pyrimidin-4-yl)piperazine-1-carboxylate,
(39) tert-butyl 4-(6-(4-chlorobenzylamino)-2-(methylsulfonyl)pyrimidin-4-yl)piperazine-1-carboxylate,
(40) tert-butyl 4-(6-(4-bromophenylamino)-2-(methylsulfonyl)pyrimidin-4-yl)piperazine-1-carboxylate,
(41) tert-butyl 4-(6-(4-iodophenylamino)-2-(methylsulfonyl)pyrimidin-4-yl)piperazine-1-carboxylate,
(42) tert-butyl 4-(6-(3,4-difluorophenylamino)-2-(methylsulfonyl)pyrimidin-4-yl)piperazine-1-carboxylate,
(43) tert-butyl 4-(2-(methylsulfonyl)-6-(3,4,5-trifluorophenylamino)pyrimidin-4-yl)piperazine-1 -carboxylate,
(44) tert-butyl 4-(2-(methylsulfonyl)-6-(3,4,5-trifluorobenzylamino)pyrimidin-4-yl)piperazine-1 -carboxylate,
(45) N-(4-fluorophenyl)-2-(methylsulfonyl)-6-(piperazin-1-yl)pyrimidin-4-amine,
(46) 2-(methylsulfonyl)-6-(piperazin-1-yl)-N-(3,4,5-trifluorophenyl)pyrimidin-4-amine,
(47) N-(3-chloro-4-fluorophenyl)-2-(methylsulfonyl)-6-(piperazin-1-yl)pyrimidin-4-amine,
(48) N-(3-bromo-4-fluorophenyl)-2-(methylsulfonyl)-6-(piperazin-1-yl)pyrimidin-4-amine,
(49) 2-(methylsulfonyl)-N⁴-(piperidin-4-yl)-N⁶-(3,4,5-trifluorophenyl)pyrimidine-4,6-diamine,
(50) N⁴-(3-chloro-4-fluorophenyl)-2-(methylsulfonyl)-N⁶-(piperidin-4-yl)pyrimidine-4,6-diamine,
(51) N⁴-(3-bromo-4-fluorophenyl)-2-(methylsulfonyl)-N⁶-(piperidin-4-yl)pyrimidine-4,6-diamine,
(52) 6-(4-aminopiperidin-1-yl)-2-(methylsulfonyl)-N-(3,4,5-trifluorophenyl)pyrimidin-4-amine,
(53) (6-(4-aminopiperidin-1-yl)-N-(3-chloro-4-fluorophenyl)-2-(methylsulfonyl)pyrimidin-4-amine,
(54) 6-(4-aminopiperidin-1 -yl)-N-(3-bromo-4-fluorophenyl)-2-(methylsulfonyl)pyrimidin-4-amine,
(55) 6-((1R,4R)-2,5-diazabicyclo[2.2.1]heptan-2-yl)-N-(3-chloro-4-fluorophenyl)-2-(methylsulfonyl)pyrimidin-4-amine,
(56) N-(3-chloro-4-fluorophenyl)-2-(methylsulfonyl)-6-(2,6-diazaspiro[3.3]heptan-2-yl)pyrimidin-4-amine,
(57) 6-((1S,4S)-2,5-diazabicyclo[2.2.1]heptan-2-yl)-N-(3-chloro-4-fluorophenyl)-2-(methylsulfonyl)pyrimidin-4-amine, or
(58) ((1R,5S,6s)-3-(6-(3-chloro-4-fluorophenylamino)-2-(methylsulfonyl)pyrimidin-4-yl)-3-azabicyclo[3.1 .0]hexan-6-amine).

8. A method for preparing a compound represented by Formula 1 or a pharmaceutically acceptable salt thereof, the method comprising :
Step 1: preparing a compound represented by Formula 4-1 by reacting a compound represented by Formula 2-1 with an amine derivative represented by Formula 3; and
optionally, when R₄ is not hydrogen, Step 2: preparing a compound represented by Formula 6 by reacting a compound represented by Formula 4-1 with a halide derivative represented by Formula 5; 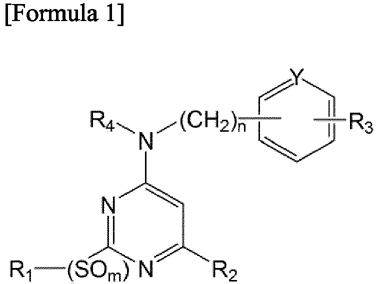 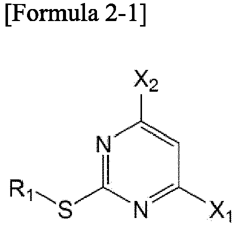 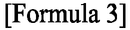 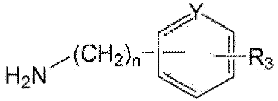 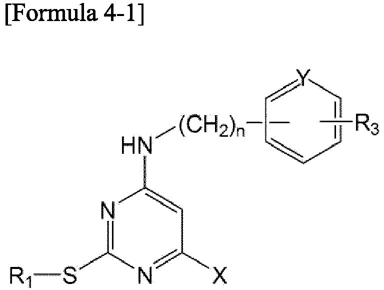
[Formula 5] R₄-X₃ 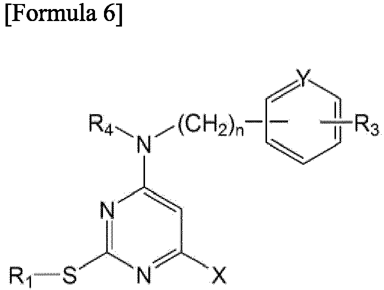
wherein,
X₁ to X₃ are each independently halogen;
X is X₁ or 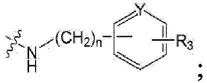
m is 0, 1, or 2;
n is an integer from 0 to 4;
Yis C(H) or N;
R₁ is C₁₋₄ alkyl;
R₂ is hydrogen, halogen, amino, -NH-(CH₂)ₚ-NHCO-R₅, or
C₆₋₁₀ aryl, C₆₋₁₀ arylamino, 3-10 membered heterocyclyl, or 3-10 membered heterocyclylamino each unsubstituted or substituted by R₆;
R₅ is tert-butoxy, C₆₋₁₀ aryl or 5-10 membered heteroaryl;
R₆ is tert-butoxy, C₆₋₁₀ aryl or 5-10 membered heteroaryl linked directly or through - CO-;
p is an integer from 1 to 4;
R₃ is hydrogen, 3-10 membered heterocyclyl, cyano, C₁₋₄ alkyl, C₁₋₄ haloalkyl, or 1 to 3 halogens; and
R₄ is hydrogen, or C₆₋₁₀ aryl-C₁₋₄ alkyl;
wherein said aryl, heteroaryl and heterocyclyl are unsubstituted or substituted by one or more selected from halogen, C₁₋₄ alkyl, C₁₋₄ alkoxy, or amino.

9. The method of claim 8,
further comprising Step 3-1: preparing a compound represented by Formula 7 by reacting a compound represented by Formula 6 with ammonium hydroxide; 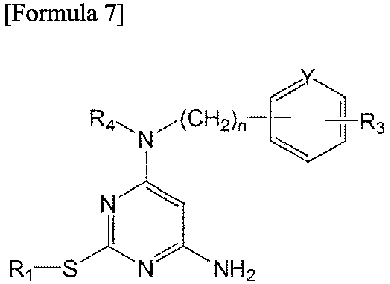 wherein,
n is an integer from 0 to 4;
Yis C(H) or N;
R₁ is C₁₋₄ alkyl;
R₃ is hydrogen, 3-10 membered heterocyclyl, cyano, C₁₋₄ alkyl, C₁₋₄ haloalkyl, or 1 to 3 halogens; and
R₄ is hydrogen or C₆₋₁₀ aryl-C₁₋₄ alkyl;
wherein said aryl, heteroaryl and heterocyclyl are unsubstituted or substituted by one or more selected from halogen, C₁₋₄ alkyl, C₁₋₄ alkoxy, or amino.

10. The method of claim 8, further comprising
Step 3-2: preparing a compound represented by Formulas 16 or 23, respectively, by reacting a compound represented by Formula 6 with a Boc-protected diamine derivative represented by any one of Formulas 8 to 15 ; and
Step 4-1: preparing a compound represented by Formulas 25 or 26, respectively, by reacting a compound represented by Formula 16 or 17 with an acyl halide derivative represented by Formulas 24, or Step 4-2: preparing a compound represented by Formulas 27 to 33 by oxidizing an alkylthio group of a compound represented by Formula 17 to 23 to an alkylsulfonyl group and deprotecting it;
[Formula 8] NH₂-(CH₂)ₚ-NH-Boc 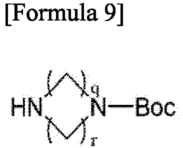 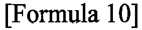 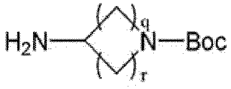 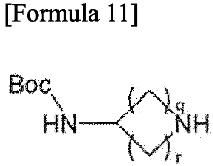 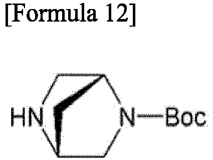 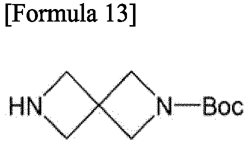 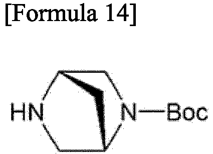 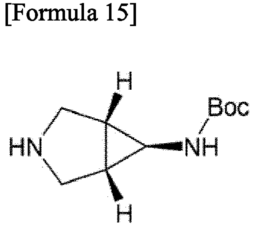 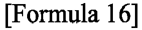 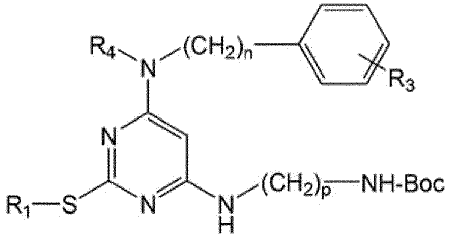 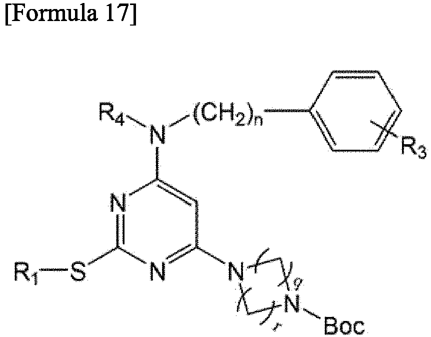 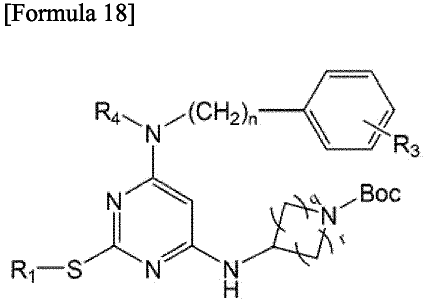 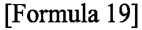 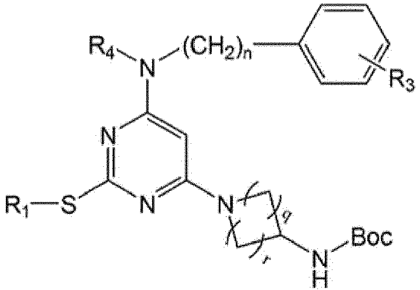 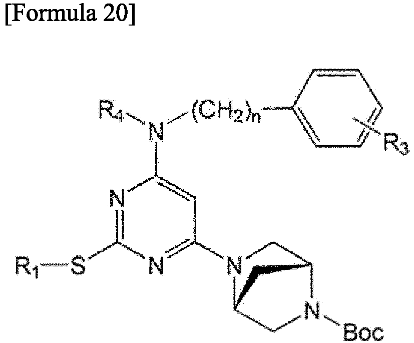 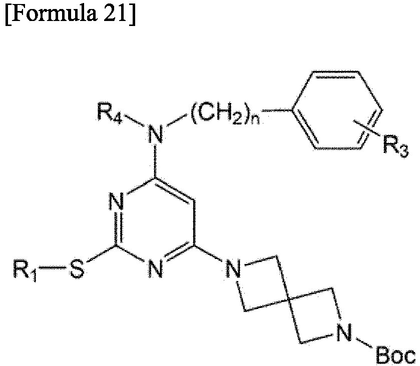 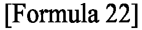 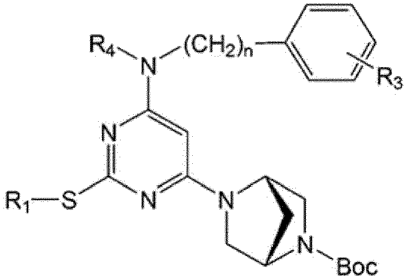 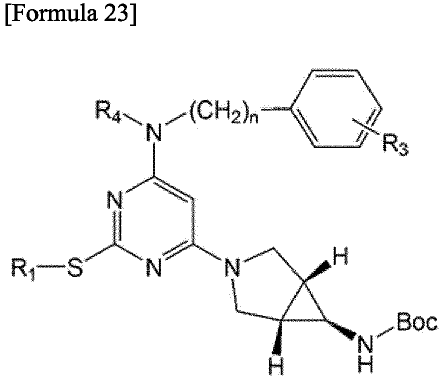
[Formula 24] R-COX₄ 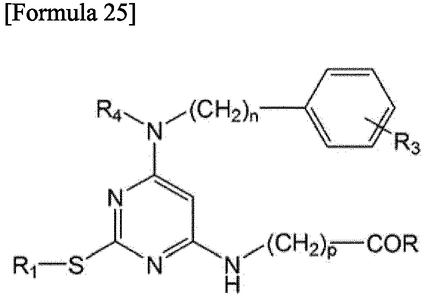 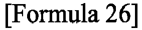 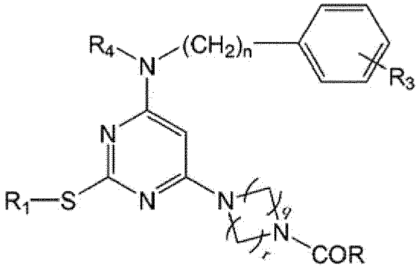 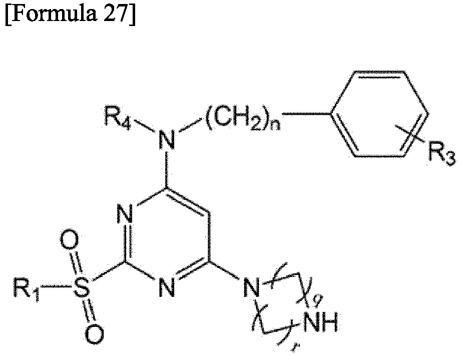 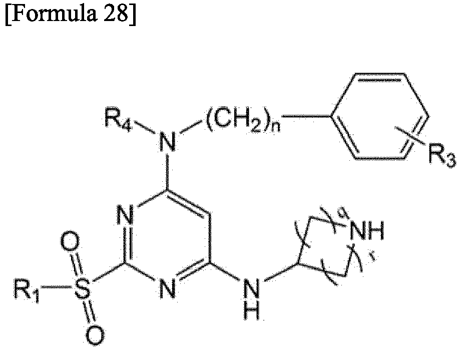 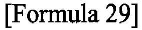 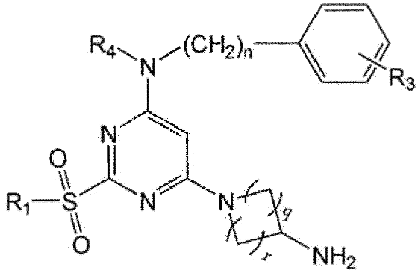 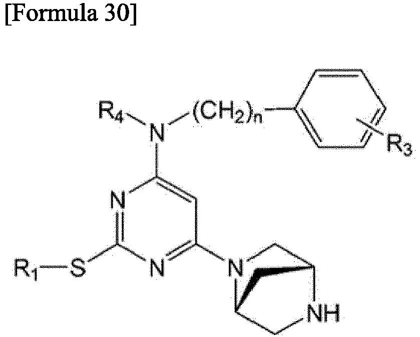 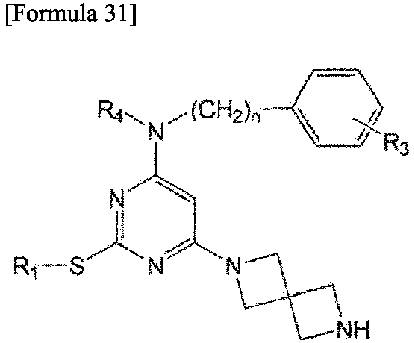 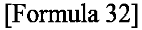 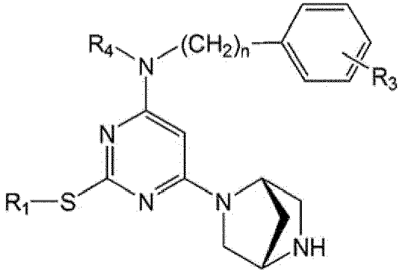 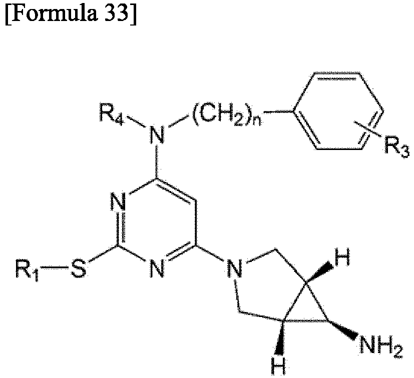
wherein,
n is an integer from 0 to 4;
p is an integer from 1 to 4;
q is 0, 1 or 2;
r is 1 or 2;
X₄ is halogen;
R₁ is C₁₋₄ alkyl;
R₃ is hydrogen, 3-10 membered heterocyclyl, cyano, C₁₋₄ alkyl, C₁₋₄ haloalkyl, or 1 to 3 halogens;
R₄ is hydrogen, or C₆₋₁₀ aryl-C₁₋₄ alkyl; and
R is C₆₋₁₀ aryl or 5-10 membered heteroaryl;
whrein said aryl, heteroaryl and heterocyclyl are unsubstituted or substituted by one or more selected from halogen, C₁₋₄ alkyl, C₁₋₄ alkoxy or amino.

11. The method of any one of claims 8 to 10,
further comprising Step 5: oxidizing an alkylthio group to a sulfinyl group or a sulfonyl group by reacting it with mCPBA.

12. A method for preparing a compound represented by Formula 1 or a pharmaceutically acceptable salt thereof, the method comprising :
Step 1: preparing a compound represented by Formula 4 by reacting a compound represented by Formula 2-2 with an amine derivative represented by Formula 3; and
Step 2: preparing a compound a compound represented by Formula 34 by reacting a compound represented by Formula 4-2 with a Boc-protected diamine derivative represented by Formula 9; 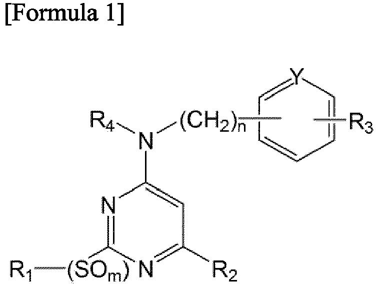 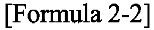 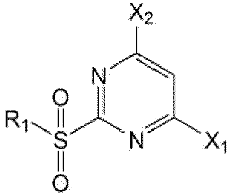 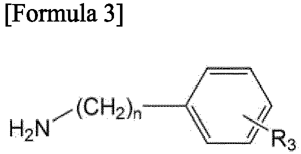 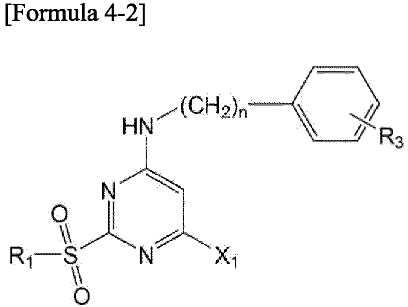 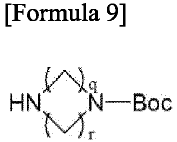 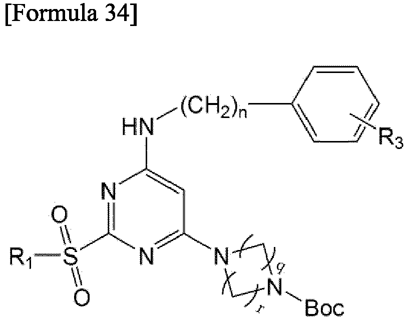 wherein,
X₁ and X₂ are each independently halogen;
m is 2;
n is an integer from 0 to 4;
R₁ is C₁₋₄ alkyl;
R₂ is 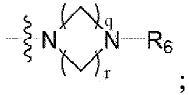 R₆ is -CO-tert-butoxy;
q is 0, 1 or 2;
r is 1 or 2;
R₃ is hydrogen, 3-10 membered heterocyclyl, cyano, C₁₋₄ alkyl, C₁₋₄ haloalkyl or 1 to 3 halogens; and
R₄ is hydrogen;
wherein said heterocyclyl is unsubstituted or substituted by one or more selected from halogen or amino.

13. A composition for inhibiting capsid assembly comprising a compound of any one of claims 1 to 7 or a pharmaceutically acceptable salt thereof.

14. An antiviral composition comprising a compound of any one of claims 1 to 7 or a pharmaceutically acceptable salt thereof as an active ingredient.

15. A pharmaceutical composition for preventing or treating a viral infectious disease comprising a compound of any one of claims 1 to 7 or a pharmaceutically acceptable salt thereof as an active ingredient.

16. A pharmaceutical composition of claim 15,
wherein the viral infectious disease is an infectious disease caused by hepatitis B virus (HBV), hepatitis C virus (HCV) or human immunodeficiency virus (HIV).
